# EUROPEAN PATENT APPLICATION

(11) **EP 4 000 627 A1**
(43) Date of publication of application: **25.05.2022**
(21) Application number: 20382991.6
(22) Date of filing: 17.11.2020
(51) Int. Cl.: A61K 38/00, A61K 38/08, C07K 14/47, C12N 9/12, A61P 43/00, A61P 9/00

(54) **TREATMENT OF ABDOMINAL AORTIC ANEURYSM AFTER ITS CLINICAL MANIFESTATION**

(71) Applicant: Fundacion Instituto De Investigacion Sanitaria Fundacion Jimenez Diaz, 28040 Madrid (ES); Marasco, Daniela, 80147 Naples (IT); La Manna, Sara, 80053 Naples (IT)
(72) Inventor: MARASCO, Daniela, 80147 Naples (IT); LA MANNA, Sara, 80053 Naples (IT); GÓMEZ GUERRERO, Carmen, 28040 Madrid (ES); EGIDO DE LOS RÍOS, Jesús, 28040 Madrid (ES); BERNAL URIBE, Susana, 28040 Madrid (ES); LÓPEZ SANZ, Laura, 28040 Madrid (ES)
(74) Representative: Hoffmann Eitle

(57) **Abstract**

The present invention refers to the treatment of abdominal aortic (AAA) aneurysm after its clinical manifestation by using synthetic and cell penetrating peptides mimic of SOCS1.

## Description

### FIELD OF THE INVENTION

The present invention refers to the medical filed. Particularly, it refers to the treatment of abdominal aortic (AAA) aneurysm after its clinical manifestation, by using synthetic and cell penetrating peptides mimic of suppressor of cytokine signalling-1 (SOCS1) protein.

### PRIOR ART

AAA is a chronic vascular disorder characterized by transmural aortic wall degeneration between the diaphragm and iliac bifurcation, leading to progressive dilation and weakening of the abdominal aorta.

Pathological mechanisms of AAA development include protease-mediated degradation of extracellular matrix (ECM) components (e.g. collagen and elastin fibres), dysfunction of vascular smooth muscle cells (VSMC), enhanced leukocyte infiltration, mechanical stress and reactive oxygen species (ROS) production in the vessel wall. Chronic inflammation is an important feature of AAA, with presence of T and B lymphocytes, neutrophils and monocytes/macrophages in the aneurysmal tissue. During vascular remodelling, inflammatory cells secrete ECM degrading enzymes such as metalloproteinase (MMP) 2, 3 and 9 that results in progressive destruction of structural proteins, and eventual rupture of the vessel. The secretion of proinflammatory cytokines (e.g., interleukin (IL)-1, IL-6, tumour necrosis factor-α (TNFα), TNF-like weak inducer of apoptosis, and interferon-a (IFN-α)) and chemokines (e.g. C-C motif chemokine ligand (CCL) 2 and 5) from infiltrating or resident cells also contributes to vascular inflammation and aortic damage.

Several researchers have investigated the gene expression profile of human and experimental AAA, and identified multiple genes and pathways involved in the pathogenesis of AAA. Among them, cytokine-receptor interaction has been shown to induce a complex network of inflammatory signalling pathways, including nuclear factor-kB, mitogen-activated protein kinase kinases, and the Janus kinase/Signal transducers and activator of transcription (JAK/STAT). Overactivation of these pathways in aneurysmal tissue promotes phenotypic changes of T lymphocytes, macrophages and VSMC, and upregulates the expression of genes involved in ECM degradation, oxidative stress and cell migration, further exacerbating inflammation and tissue injury.

Cytokine signalling depending on the Janus kinase/signal transducer and activator of transcription (JAK/STAT) pathway regulates the expression of many genes involved in activation, differentiation, migration, apoptosis and proliferation, and is a major contributor to chronic inflammatory diseases. Four kinases (JAK1-3 and TYK2) and seven transcription factors (STAT1-4, 5A, 5B and 6) compose the JAK/STAT family, which are controlled in a classical negative-feedback loop by the suppressors of cytokine signalling (SOCS) family (SOCS1-7 and CIS). JAK/STAT pathway is a critical regulator of inflammatory processes in various cells of the cardiovascular system, including endothelial cells, VSMC and inflammatory cells. Moreover, clinical and experimental evidence implicates enhanced expression/activation of JAK/STAT members and regulated genes in AAA. Because JAK/STAT/SOCS axis may offer therapeutic intervention opportunities to be explored in cardiovascular diseases, this work investigates whether a SOCS1-based therapy limits AAA development. To that end, the effects of a cell-permeable peptide spanning the kinase inhibitory region (KIR) of SOCS1 protein to disrupt JAK/STAT activation were analysed in elastase perfusion-induced AAA in mice, an experimental model with multiple similarities to human aneurysm including medial elastin degradation and adventitial inflammatory cell infiltration. *In vivo* studies are complemented by mechanistic studies in VSMC and macrophages stimulated with elastase and elastin fragments.

Although generally asymptomatic, AAA progression over time increases the risk of rupture causing acute haemorrhage and high mortality when surgical treatment is unsuitable. Therapeutic strategy for patients with AAA is limited to open or endovascular surgical therapy of large AAA (>5.5cm in diameter) which is not applicable for small AAA.

The current lack of effective pharmacotherapies for AAA underlies the huge need for translational, mechanism-driven research aimed at therapeutic targets to halt the growth and rupture of AAA or to delay surgical repair.

The present invention is aimed at solving this problem and a new therapeutic approach, based on the use of synthetic and cell penetrating peptides mimic of SOCS1, is herein proposed for the treatment of AAA after its clinical manifestation.

### DESCRIPTION OF THE INVENTION

### Brief description of the invention

As explained above, AAA is a multifactorial disease characterized by chronic inflammation, oxidative stress, and proteolytic activity in the aortic wall. The inventors of the present invention have investigated the vasculoprotective role of suppressor of cytokine signalling-1 (SOCS1), the negative JAK/STAT regulator, in experimental AAA.

Precisely, synthetic, cell penetrating peptides mimic of SOCS1 kinase inhibitory domain to suppress STAT activation was assayed both *in vitro* and in the well-established mouse model of elastase-induced AAA by monitoring changes in aortic diameter, cellular composition, and gene expression in abdominal aorta. The function of the peptides was further evaluated in cultured vascular smooth muscle cells (VSMC) and macrophages exposed to elastase, elastin-derived peptides or a combination of proinflammatory cytokines (IFNγ and IL-6).

The peptides assayed in the present invention prevented AAA development after its clinical manifestation, evidenced by reduced incidence of AAA, aortic dilation and elastin degradation, partial restoration of medial VSMC, and decreased inflammatory cells and oxidative stress in AAA tissue. Mechanistically, the peptides of the invention suppressed STAT1/3 activation in aorta, downregulated cytokines and metalloproteinases, and altered the expression of cell differentiation markers by favouring anti-inflammatory M2 macrophage and contractile VSMC phenotypes. *In vitro*, the peptides of the invention suppressed the expression of inflammatory and oxidative genes, reduced cell migration, and reversed the phenotypic switch of macrophages and VSMC. By contrast, SOCS1 silencing promoted inflammatory response.

In conclusion, the present invention demonstrates the therapeutic potential of SOCS1-derived peptides to halt AAA progression by suppressing JAK/STAT-mediated inflammation and aortic dilation.

So, the first embodiment of the present invention refers to an isolated peptide comprising an inhibitory motif of JAK2 consisting of the Formula I **DT*X₁X₂X₃*T*X₄*R*X₅*H** (I) wherein: **D** is Aspartic acid, **T** is Threonine, ***X₁*** is selected between Histidine or S-acetaminomethyl-L-cysteine (*C_{Acm}*), ***X₂*** is selected between Phenylalanine or Arginine, ***X₃*** is selected between Arginine or Glutamine, **T** is Threonine, ***X₄*** is Phenylalanine or 1-naphthylalanine (*Nal₁*), **R** is Arginine, ***X₅*** is selected between Serine or Lysine, with the proviso that when ***X₅*** is Lysine a lactam bridge is formed with the Aspartic acid to create a cyclic peptide, and **H** is Histidine, for use in the treatment of abdominal aortic aneurysm after its clinical manifestation.

In a preferred embodiment, the peptide comprises the inhibitory motif of JAK2 which consists of SEQ ID NO: 1 (DTHFRTFRSH), SEQ ID NO: 2 (DT*C_{Acm}*RQTFRSH), SEQ ID NO: 3 (DT*C*_{*A*cm}RQT*Nal₁*RSH), SEQ ID NO: 4 (DT*C_{Acm}*RQTFRKH) or SEQ ID NO: 5 (DT*C_{Acm}*RQT*Nal₁*RKH).

In a preferred embodiment, the peptide which comprises the inhibitory motif of JAK2 consisting of SEQ ID NO: 1 further comprises an adjacent peptide fragment at the C-terminus consisting of the SEQ ID NO: 6 (ADYRRI).

In a preferred embodiment, the peptide comprises the SEQ ID NO: 7 (DTHFRTFRSHADYRRI).

In a preferred embodiment, the peptide further comprises a cell penetrating peptide at the N-terminus.

In a preferred embodiment, the peptide which comprises the SEQ ID NO: 7, further comprises a cell penetrating peptide consisting of palmitoyl-lysine at the N-terminus.

In a preferred embodiment, the peptide consists of the palmitoyl-SEQ ID NO: 8 (KDTHFRTFRSHADYRRI) (also named as "S1 peptide") or a peptide having at least 85% of identity with the SEQ ID NO: 8.

In a preferred embodiment, the peptide which comprises the SEQ ID NO: 2, SEQ ID NO: 3, SEQ ID NO: 4 or SEQ ID NO: 5 further comprises a cell penetrating peptide consisting of SEQ ID NO: 9 (GRKKRRQRRRPPQGG).

In a preferred embodiment, the peptide consists of the:
- SEQ ID NO: 10 GRKKRRQRRRPPQGGDT*C_{Acm}*RQTFRSH (also named as "linear S1PS5"),
- SEQ ID NO: 11 GRKKRRQRRRPPQGGDT*C_{Acm}*RQT*Nal₁*RSH (also named as "linear S1PS5 Nal1"),
- SEQ ID NO: 12 GRKKRRQRRRPPQGGDT*C_{Acm}*RQTFRKH (also named as "internal cyclic S1PS5"), wherein a lactam bridge is formed between the Lysine and the Aspartic acid to create a cyclic peptide.
- SEQ ID NO: 13 GRKKRRQRRRPPQGGDT*C_{Acm}*RQT*Nal₁*RKH (also named as "internal cyclic S1PS5 Nal1"), wherein a lactam bridge is formed between the Lysine and the Aspartic acid to create a cyclic peptide.
or a peptide having at least 85% of identity with the SEQ ID NO: 10, SEQ ID NO: 11, SEQ ID NO: 12 or SEQ ID NO: 13.

In a preferred embodiment, the peptide is administered by intraperitoneal or subcutaneous administration.

The second embodiment refers to an isolated peptide consisting of the SEQ ID NO: 10, SEQ ID NO: 11, SEQ ID NO: 12, SEQ ID NO: 13, or a peptide having at least 85% of identity with the SEQ ID NO: 10, SEQ ID NO: 11, SEQ ID NO: 12 or SEQ ID NO: 13, wherein, in the case of peptides with SEQ ID NO: 12 or 13 a lactam bridge is formed between the Lysine and the Aspartic acid to create a cyclic peptide.

The third embodiment of the present invention refers to the above cited peptides of SEQ ID NO: 10, 11, 12 or 13 for use as medicament.

The fourth embodiment of the present invention refers to a pharmaceutical composition comprising an isolated peptide of SEQ ID NO: 10, 11, 12 or 13 and, optionally, pharmaceutically acceptable excipients or carriers.

The fifth embodiment of the present invention refers to a method for treating AAA after its clinical manifestation which comprises administering a therapeutically effective dose or amount of a peptide comprising an inhibitory motif of JAK2 consisting of the Formula I, preferably a peptide consisting palmitoyl-SEQ ID NO: 8, SEQ ID NO: 10, SEQ ID NO:11, SEQ ID NO:12 or SEQ ID NO:13 or a peptide having at least 85% of identity with the SEQ ID NO: 8, SEQ ID NO: 10, SEQ ID NO:11, SEQ ID NO:12 or SEQ ID NO:13.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included with the cell suspension of the invention and that causes no significant adverse toxicological effects to the patient.
- By "therapeutically effective dose or amount" of a composition comprising the peptide of the invention is intended an amount that, when administered as described herein, brings about a positive therapeutic response in a subject having AAA. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.
- By "inhibitory motif of JAK2" it meant a sequence region that inhibits JAK tyrosine kinase activity by binding to the activation loop site.
- Finally, the following non-standard abbreviations are herein included: ACTA2, actin-α2; ARG, arginase; BMDM, bone marrow-derived macrophages; CD206, macrophage mannose receptor-1; CCL, C-C motif chemokine ligand; CXCL, C-X-C motif chemokine ligand; ECM, extracellular matrix; EDP, elastin-derived peptide; FLF4, Krueppel-like factor-4; iNOS, inducible nitric oxide synthase; MTT, 3-(4,5-dimethylthiazol-2-yl)-2,5-diphenyltetrazolium bromide tetrazolium; NOX, NADPH oxidase; siRNA, small interfering RNA; SM22, smooth muscle protein 22-α; SOCS, suppressor of cytokine signalling; S1, SOCS1 mimetic peptide; STAT, signal transducer and activator of transcription; TIMP, tissue inhibitor of metalloproteinase; VSMC, vascular smooth muscle cells.

### Description of the figures

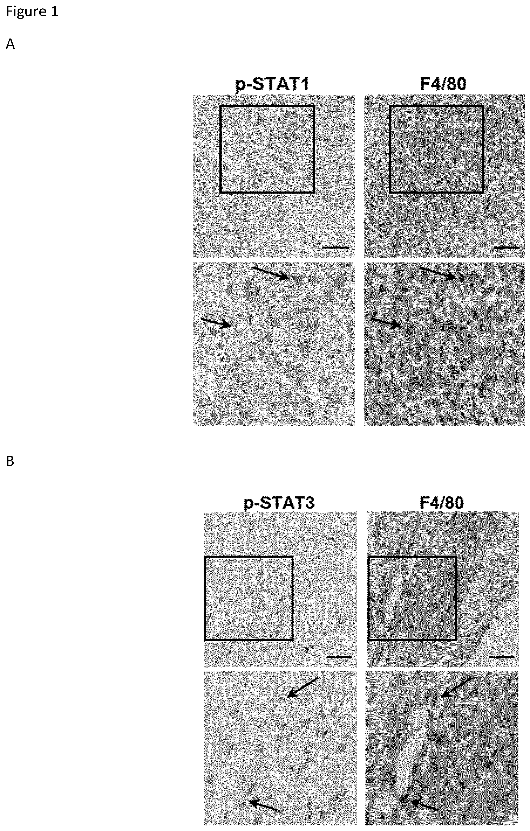

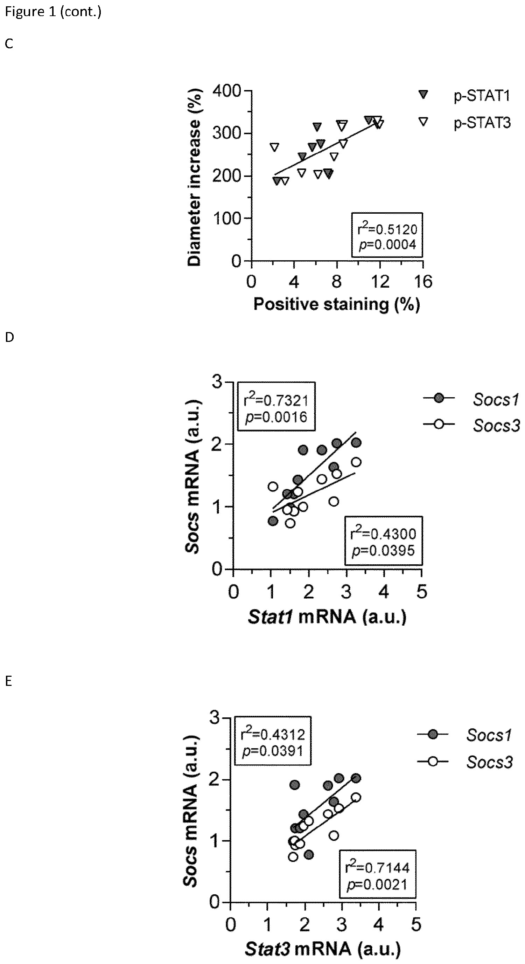

**Figure 1****. JAK/STAT pathway activation in experimental AAA. (A-B)** Immunohistochemical detection of p-STAT1 **(A)** and p-STAT3 **(B)** and colocalization with macrophages (F4/80) in aortic sections from the elastase-induced AAA mouse model. Shown are representative images (scale bars, 50 µm) and expanded views of the rectangular areas. Arrows indicate positive cells. **(C)** Pearson's correlation analysis of p-STAT1 and p-STAT3 positive immunostaining (expressed as percentage of total area) with the aortic diameter increment in AAA lesions. Positive correlation of *Socs1*/*3* expression levels with *Stat1* **(D)** and *Stat3* **(E)** in aortic tissue from AAA model (n=10 mice). Real-time PCR values normalised to 18S rRNA are expressed as arbitrary units (a.u.). Pearson's r and *p* values for the correlations are given within the figures.

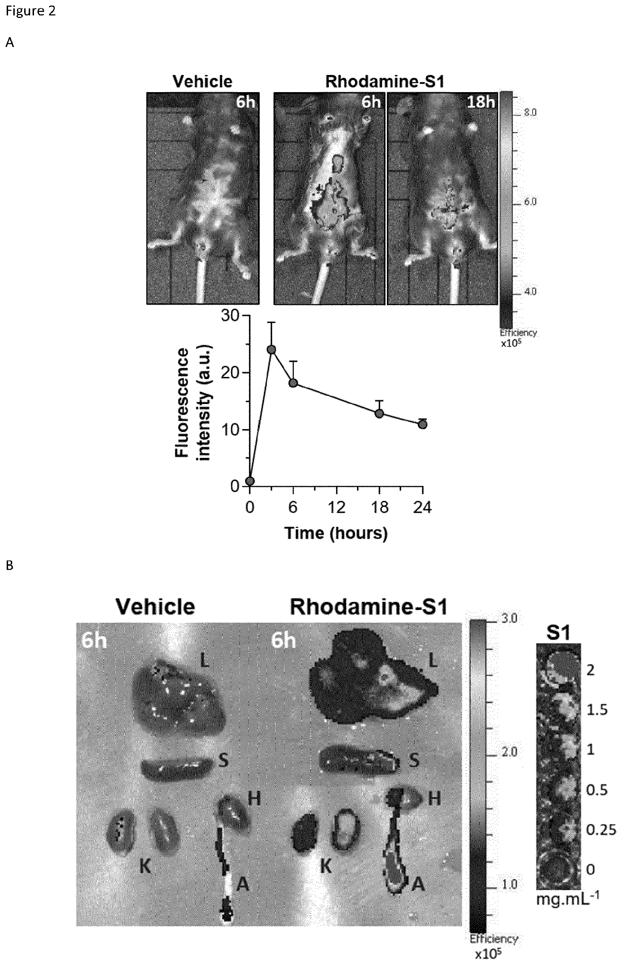

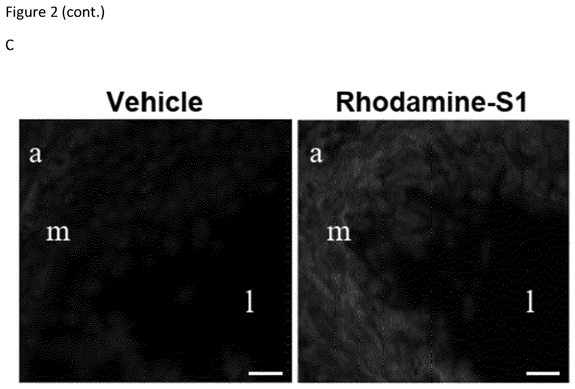

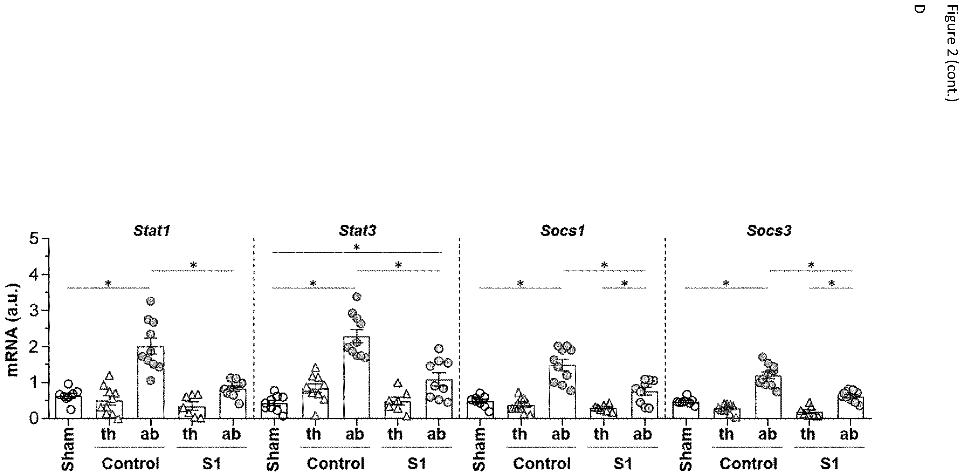

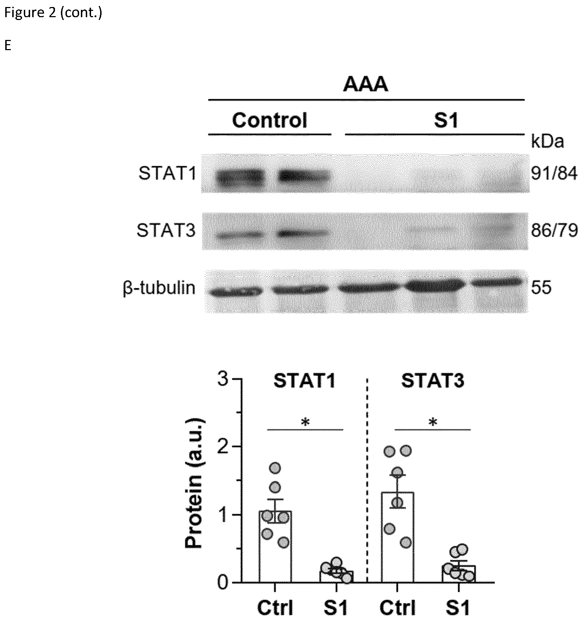

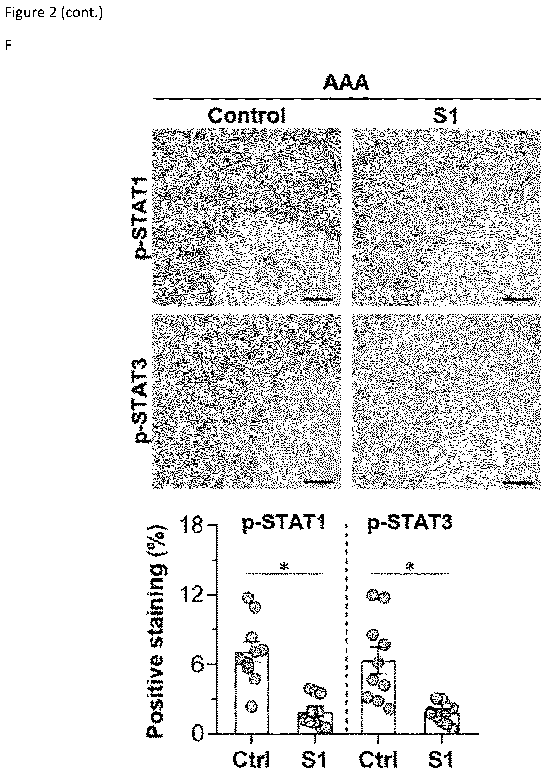

**Figure 2****. *In vivo* targeting of JAK/STAT pathway in experimental AAA. (A-C)** Biodistribution of S1 peptide in the AAA mouse model. Seven days after elastase perfusion, rhodamine labelled S1 peptide was injected intraperitoneally and allowed to target for 3, 6, 18 and 24 hours. **(A)** Representative *in vivo* IVIS images of whole mouse after S1 injection (6 and 18 h) and summary of relative fluorescence intensities along the time (n=3 animals). Statistical analysis was not performed on these data. **(B)** *Ex vivo* imaging of mouse tissues at 6 hours of S1 injection, and *in vitro* fluorescence signal of serial dilutions of peptide in 96-well plates. (C) Representative confocal images (scale bar, 25µm) showing distribution of fluorescent S1 peptide in abdominal aortic sections at 6 hours post-injection. The orientation of aorta is described by 1, lumen; m, media; a, adventitia. **(D)** Quantitative real-time PCR analysis of STAT1/3 and SOCS1/3 mRNA expression in thoracic (th) and abdominal (ab) aortic tissue from elastase-perfused mice (Control, n=10; S1, n=9), using abdominal aorta from saline-perfused mice (Sham, n=8) as reference group. PCR values normalised to 18S rRNA are expressed as arbitrary units (a.u.). **(E)** Western blot analysis of STAT1/3 proteins and β-tubulin (loading control) in mouse abdominal aorta from elastase-perfused mice after 14 days of treatment with either vehicle (Control; Ctrl, n=6) or SOCS1-derived peptide (S1, n=6). Representative blots and summary of normalised quantification expressed in arbitrary units (a.u.) are shwon. **(F)** Immunodetection of p-STAT1/3 proteins in AAA tissue sections from Control (Ctrl, n=10) and SOCS1-treated mice (S1, n=10). Shown are representative images (scale bar, 50µm) and the quantification of positive staining. Results in **D-F** are presented as individual data points and mean±SEM of total number of animals per group. **p*<0.05 according to one-way ANOVA plus Bonferroni **(D)** and two-tailed Student's **(E** and **F)** test.

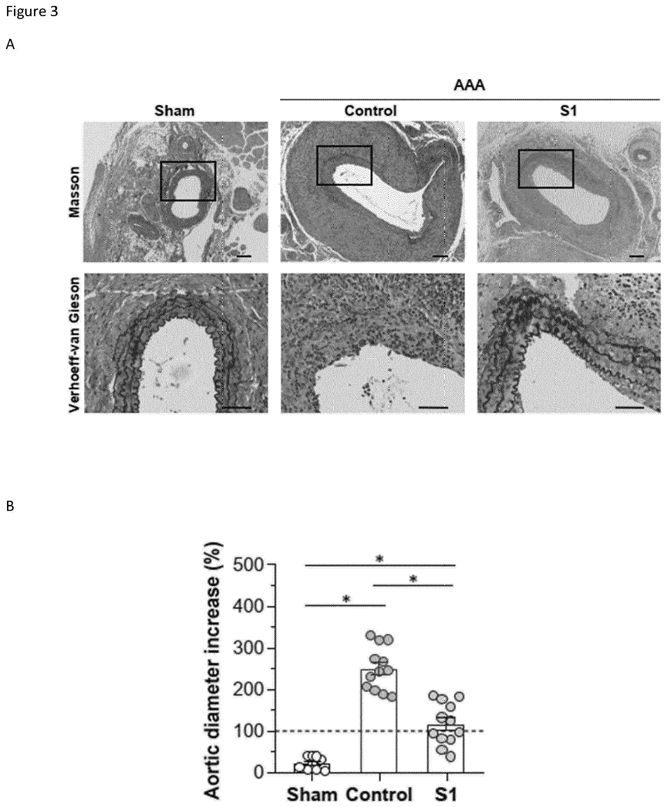

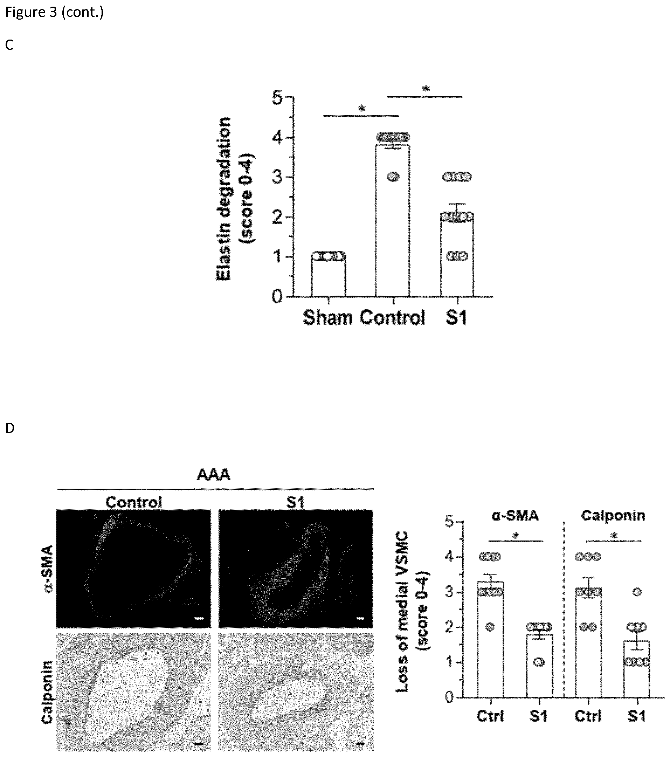

**Figure 3****. S1 peptide administration reduces elastase-induced AAA formation in mice. (A)** Representative images (scale bars, 100µm) of Masson's trichrome staining and Verhoeff-van Gieson (high-magnification fields of rectangular areas) in aortic sections from Sham (n=10), Control (n=12) and S1 (n=12) mice at 14 days postperfusion. **(B)** Quantification of the aortic diameter increase in Masson's trichrome stained lesions. **(C)** Grading of medial elastin degradation (score 0-4). **(D)** Representative immunohistochemical staining (scale bars, 100µm) and grading of VSMC contractile proteins (score 0-4) in aortic sections from elastase-perfused mice (a-SMA, n=10 per group; Calponin, n=8 per group). Results are presented as individual data points and mean±SEM of the mouse groups. **p*<0.05 according to one-way ANOVA plus Bonferroni test.

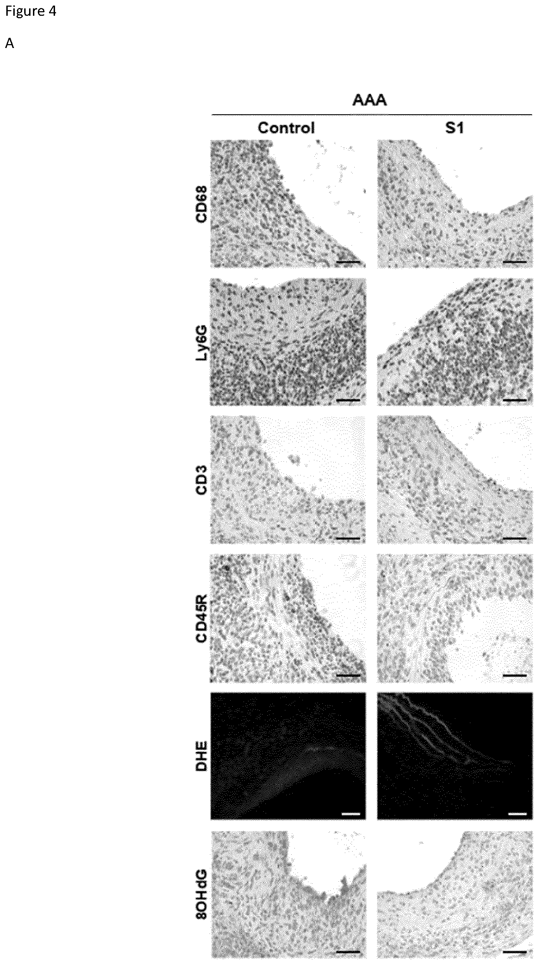

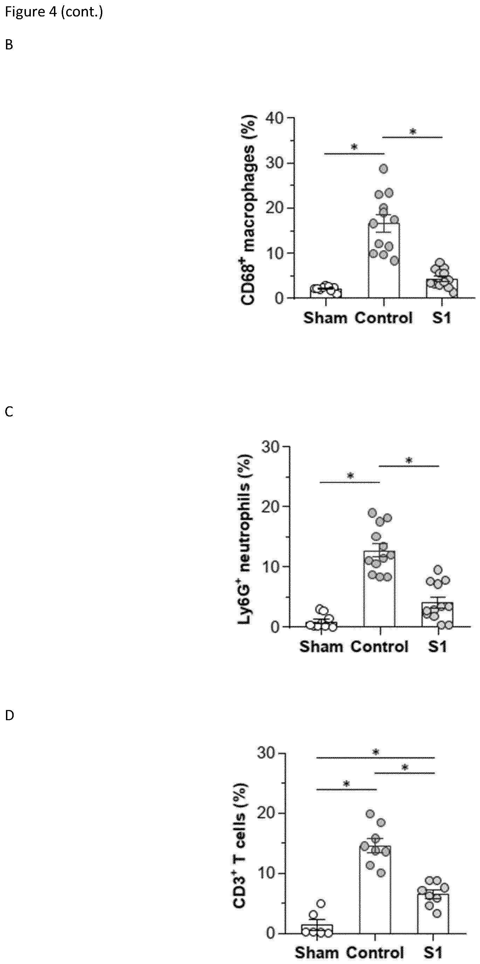

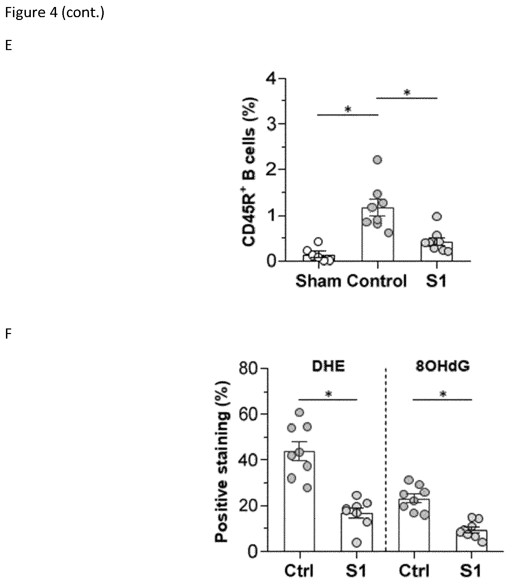

**Figure 4****. Reduced inflammatory cells and oxidative stress markers in AAA lesions from S1-treated mice. (A)** Representative images (scale bars, 50µm) of leukocytes (CD68, Ly6G, CD3 and CD45R immunoperoxidase), superoxide anion (DHE fluorescence) and DNA oxidative stress marker (8OHdG immunoperoxidase) in AAA lesions from Control and S1 treated mice after 14 days of elastase perfusion. **(B)** Quantitative analysis of macrophages as % CD68⁺ cells per lesion area (Sham, n=9; Control, n=12; S1, n=12). **(C)** Quantitative analysis of neutrophils as % Ly6G⁺ cells per lesion area (Sham, n=9; Control, n=12; S1, n=12). **(D)** Quantitative analysis of T lymphocytes as % CD3⁺ cells per lesion area (Sham, n=6; Control, n=8; S1, n=8). **(E)** Quantitative analysis of B cells as % CD45R⁺ cells per lesion area (Sham, n=8; Control, n=8; S1, n=8). **(F)** Quantification of DHE and 8OHdG positive staining per lesion area (Sham, n=8; Control, n=8; S1, n=8). Results are presented as individual data points and mean±SEM of total number of animals per group. **p*<0.05 according to one-way ANOVA plus Bonferroni **(B-E)** and two-tailed Student's **(F)** test.

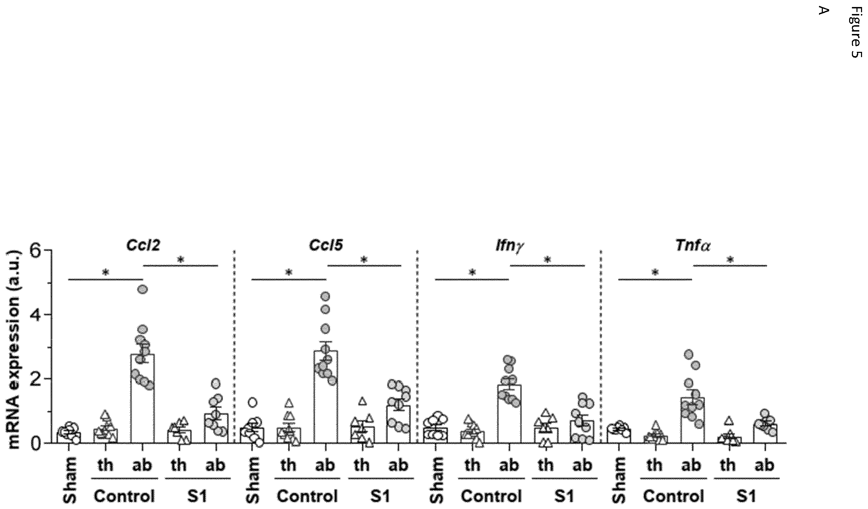

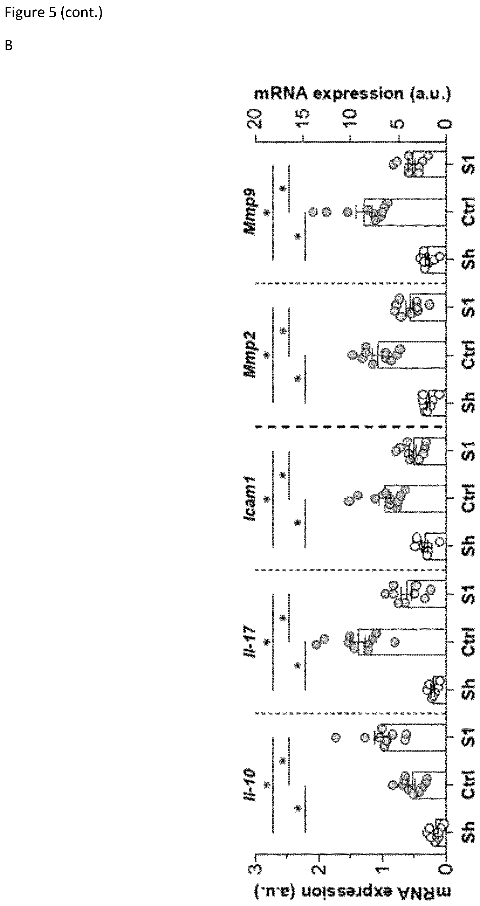

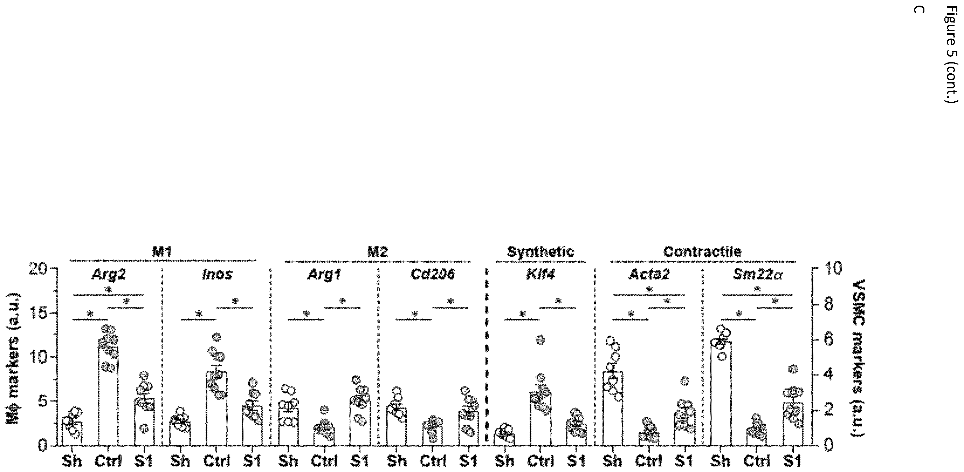

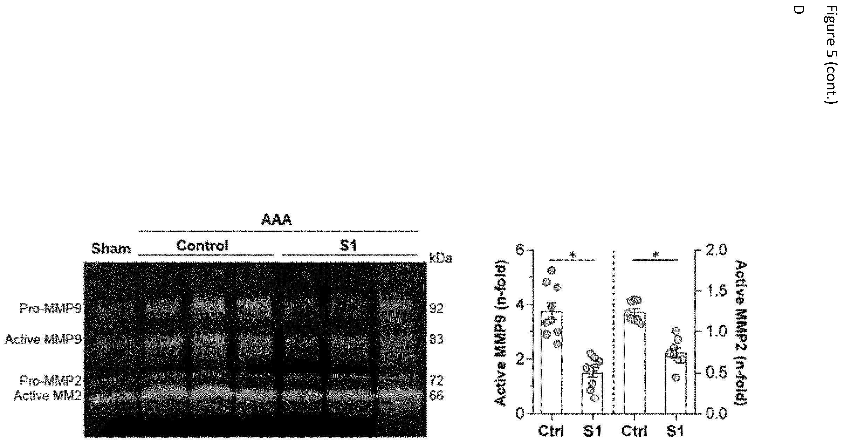

**Figure 5****. S1 peptide favourably alters the inflammatory milieu in experimental AAA. (A)** Quantitative real-time PCR analysis of chemokines and cytokines in abdominal aortic tissue from saline-perfused mice (Sham), and in thoracic (th) and abdominal (ab) aorta from elastase-perfused mice after 14 days of treatment with either vehicle (Control) or S1 peptide (S1). **(B)** Expression of pro- and antiinflammatory genes and MMPs in abdominal aorta. **(C)** Expression levels of phenotypic markers for macrophages (M1 and M2) and VSMC (synthetic and contractile) in aortic tissue. PCR values normalised to 18S rRNA are expressed as arbitrary units (a.u.). **(D)** Gelatin zymography analysis of MMPs in mouse serum samples. Shown are representative gels and densitometric analysis of active MMP9 and MMP2 relative to Sham group (arbitrarily set to 1). Results are presented as individual data points and mean±SEM of the mouse groups (Sham, n=8; Control, n=9-10; S1 n=8-9). **p*<0.05 according to one-way ANOVA plus Bonferroni **(A-C)** and non-parametric Mann-Whitney **(D)** test.

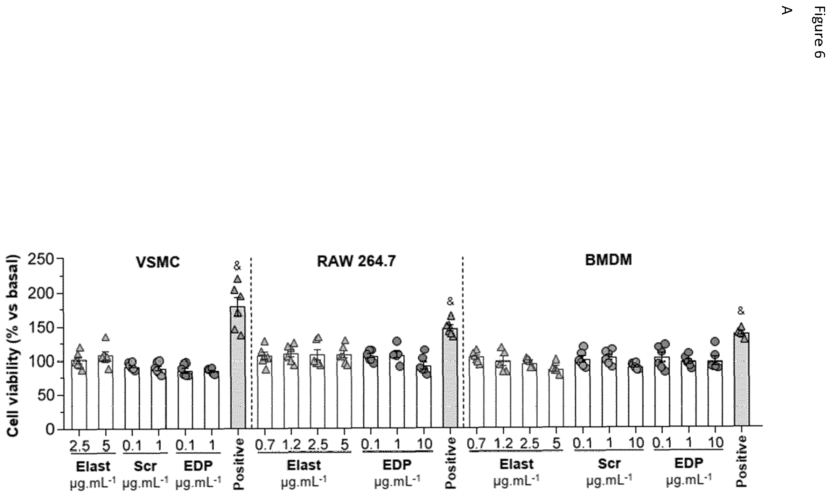

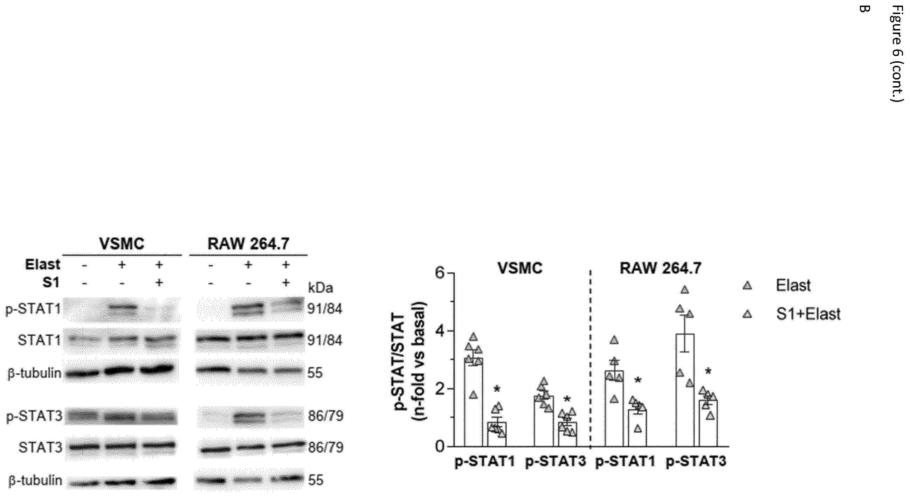

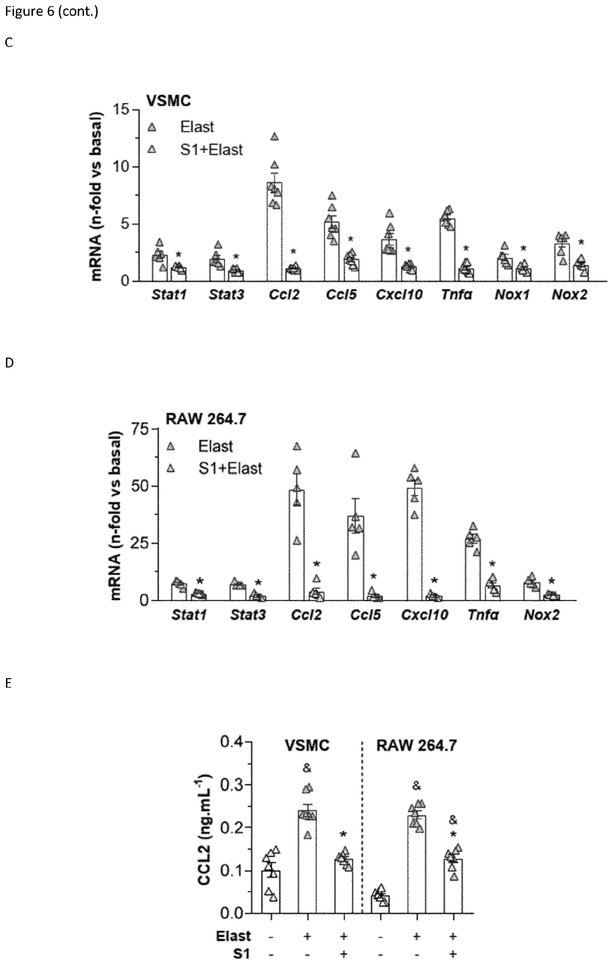

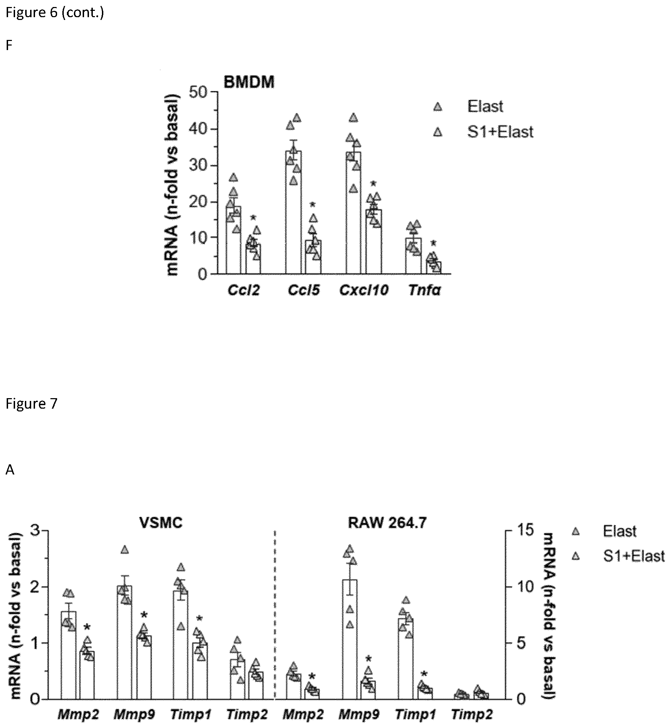

**Figure 6****. S1 peptide inhibits JAK/STAT signalling and inflammatory gene expression *in vitro.* (A)** MTT cell viability assay in mouse VSMC, RAW 264.7 macrophages and BMDM after 24 hours of incubation with different doses of elastase (Elast), scramble peptide (Scr) and elastin-derived peptide (EDP), using 10% FBS as positive control. Cell viability is expressed as percentage versus basal conditions (n=6). **(B)** VSMC and RAW 264.7 macrophages were stimulated with elastase (2.5 and 0.7 µg.mL⁻¹, respectively) in the absence or presence of S1 peptide (150 µg.mL⁻¹). Western blot analysis of phosphorylated and total STAT1/3 proteins (β-tubulin as loading control) in total cell extracts at 2 hours (VSMC, n=6) and 6 hours (RAW 264.7, n=5) of elastase stimulation. Representative blots and summary of normalised quantification are shown. **(C-D)** Gene expression of STATs, chemokines, cytokines, and Nox subunits in VSMC (24 hours, n=6-7; **C**) and macrophages (6 hours, n=5; **D**) incubated with elastase and S1 peptide. Real-time PCR values normalised by 18S endogenous control are expressed as fold increases over basal conditions (arbitrarily set to 1). **(E)** CCL2 concentrations in cell conditioned media measured by ELISA in duplicate (n=7). **(F)** Gene expression analysis in primary BMDM exposed to elastase (0.7 µg.mL⁻¹, 24 hours) in the presence or absence of S1 peptide (n=6). Results are presented as individual data points and mean±SEM of the total number of individual experiments analysed in duplicate. ^{&}*p*<0.05 versus basal and **p*<0.05 versus elastase according to one-way ANOVA plus Bonferroni **(A** and **E)** and non-parametric Mann-Whitney **(B-D** and **F)** test.

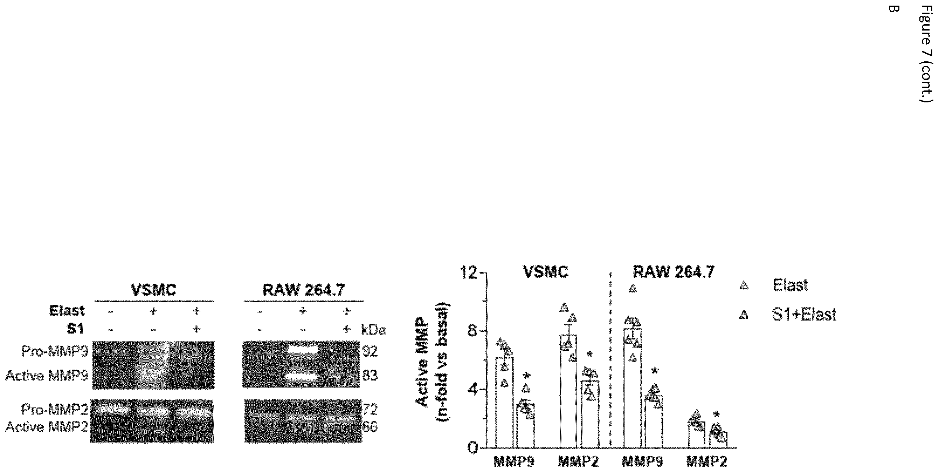

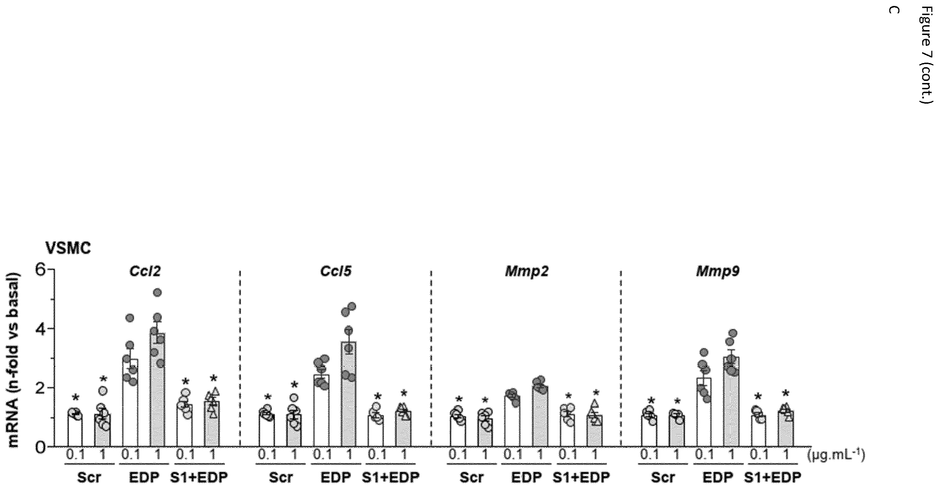

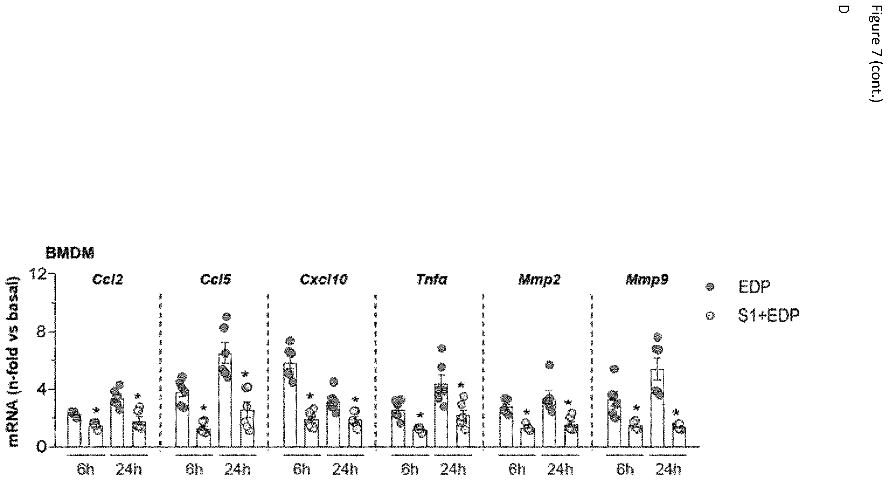

**Figure 7****. S1 peptide prevents cell responses to elastase and elastin-derived peptides. (A)** Gene expression analysis of ECM degrading enzymes and their inhibitors in VSMC and RAW 264.7 macrophages incubated with elastase (Elast; 2.5 and 0.7 µg.mL⁻¹, respectively) and 150 µg.mL⁻¹ S1 peptide (n=5). Real-time PCR values normalised by 18S endogenous control are expressed as fold increases over basal conditions (arbitrarily set to 1). (B) Gelatin zymography assay of MMPs in cell supernatants. Shown are representative gels and summary of densitometric analysis (n=5). **(C)** Gene expression analysis in VSMC after 24 hours of incubation with two doses (0.1 µg.mL⁻¹, white bars; 1 µg.mL⁻¹, grey bars) of scramble (Scr) and elastin-derived peptides (EDP) in the presence or absence of S1 peptide (n=6). **(D)** Inhibitory effect of S1 peptide on the time-dependent induction of chemokines, cytokines and MMPs in BMDM stimulated with 1 µg.mL⁻¹ EDP (n=6). Results are presented as individual data points and mean±SEM of the total number of individual experiments analysed in duplicate. **p*<0.05 versus stimulus according to Mann-Whitney **(A, B** and **D)** and Kruskal-Wallis plus Dunn's **(C)** test.

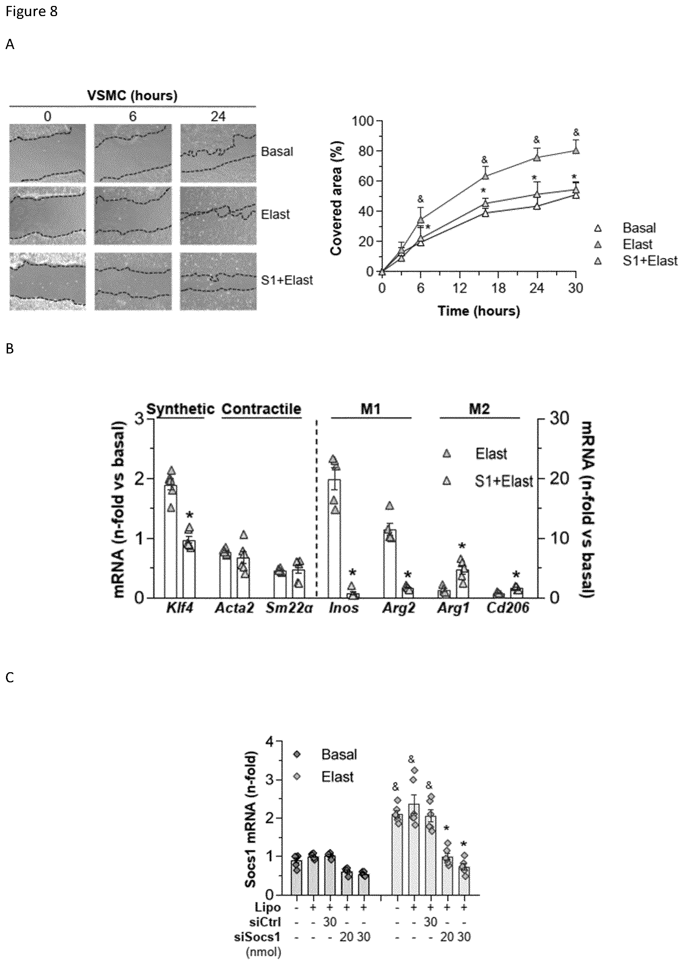

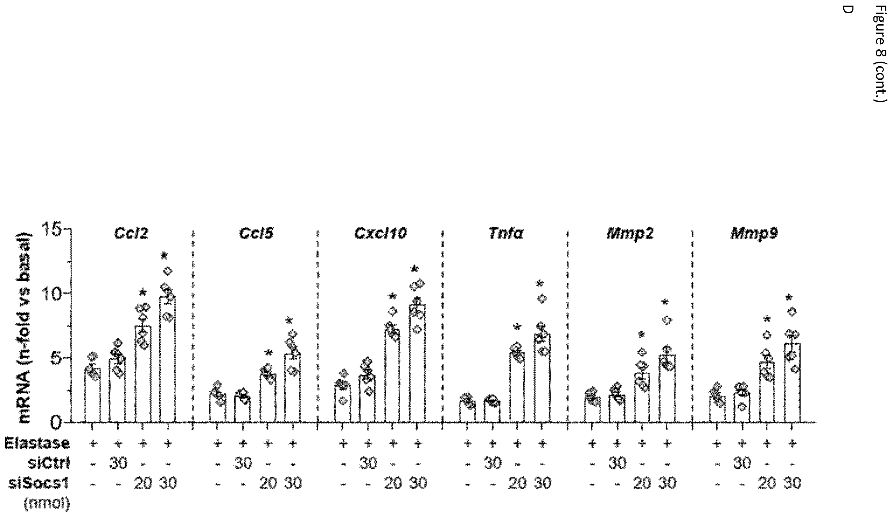

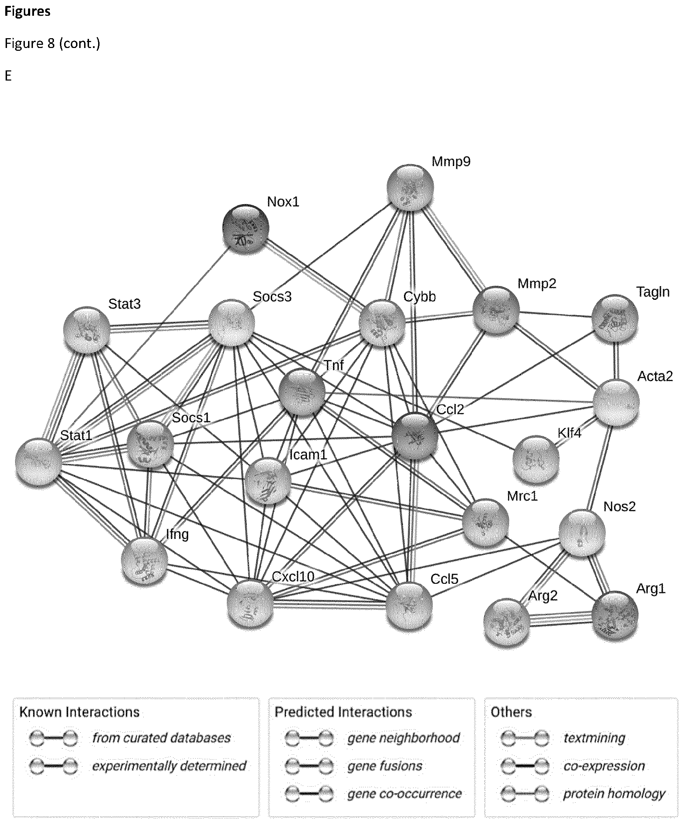

**Figure 8****. Regulatory role of SOCS1 in cell migration, polarization and inflammation. (A)** Scratch-wound-healing assay in mouse VSMC line MOVAS-1 incubated under basal conditions or elastase (2.5 µg.mL⁻¹) with/without S1 peptide. Shown are representative phase-contrast images of cells migrating into the wounded area and the quantification of covered healing areas over time, expressed as percentage versus time 0 (n=6). **(B)** Real time PCR analysis of synthetic and contractile VSMC markers (n=6) and M1 and M2 macrophage markers (n=5) at 24 hours of stimulation with elastase. **(C-D)** VSMC were transfected with SOCS1 siRNA (siSocs1), using lipofectamine alone (Lipo) and control siRNA (siCtrl) as controls, and then incubated for 24 hours without (basal) or with elastase (Elast). Gene expression levels of SOCS1 **(C)** and inflammatory genes **(D)** in SOCS1-silenced cells (n=5-6). Real time PCR values normalised by 18S endogenous control are expressed as fold increases over basal conditions (arbitrarily set to 1). ^{&}*p*<0.05 versus basal and **p*<0.05 versus elastase according to two-way ANOVA plus Tukey's **(A),** Mann-Whitney **(B)** and Kruskal-Wallis plus Dunn's **(C** and **D)** test. **(E)** STRING (https://string-db.org/) diagrams of protein-protein interactions (*Mus musculus*) of STAT1/3 and downstream genes analysed in this study. Colours of connecting lines represent different types of interactions between proteins. Cybb=Nox1, Mrc1=Cd206, Nos2=iNos, Tagln=Sm22α.

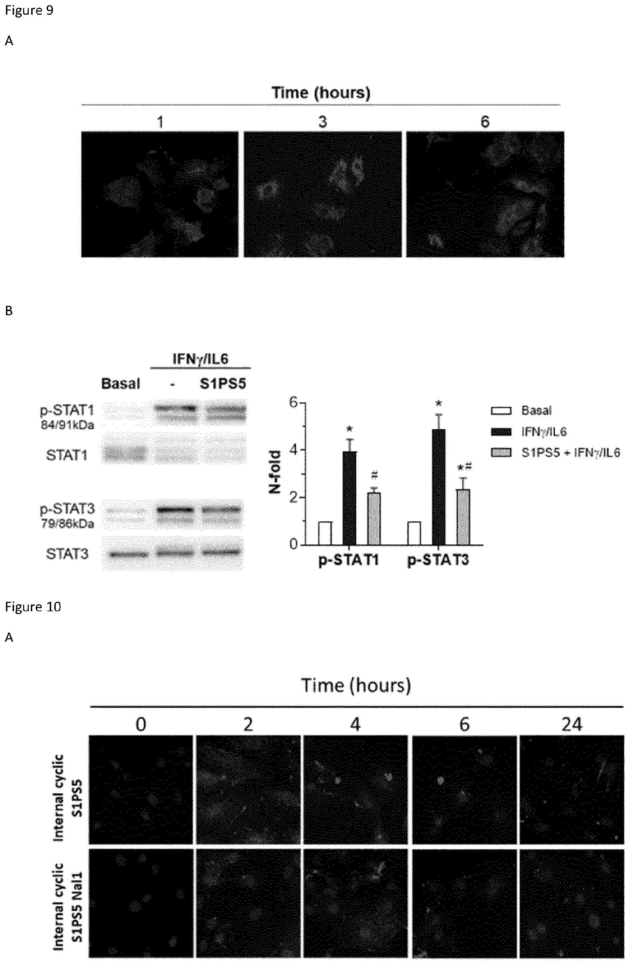

**Figure 9****. Biodistribution and functional effect of linear S1PS5 peptide *in vitro.* (A)** Confocal microscopy images showing cytosolic distribution of TAMRA-conjugated linear S1PS5 peptide in mouse VSMCs over time (blue, cell nuclei stained with DAPI; red, TAMRA-S1PS5). **(B)** Western blot analysis of phosphorylated STATs (p-STAT1 and p-STAT3) and total STAT1 and STAT3 proteins in VSMCs stimulated with a combination of inflammatory cytokines (IFNγ plus IL-6, 60 min) in the absence or presence of linear S1PS5 peptide. Shown are representative immunoblots and summary of normalized densitometry data expressed as fold increases over basal. Mean±SEM (*P<0.05 vs basal, ^{#}P<0.05 vs cytokines). Results from n=3 independent experiments.

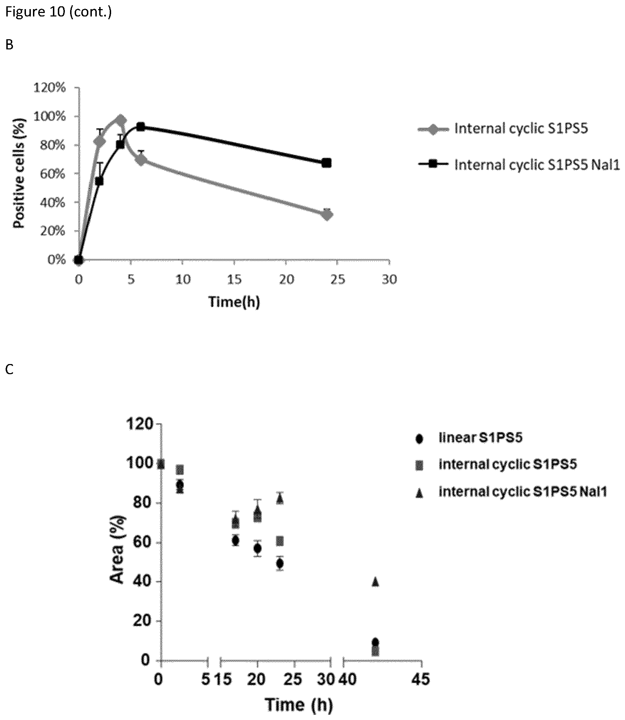

**Figure 10****. Biodistribution and serum stability of cyclic S1PS5 peptides. (A)** Representative fluorescent confocal images showing cytosolic distribution of TAMRA-conjugated internal cyclic S1PS5 peptides in mouse VSMC over time (blue, cell nuclei stained with DAPI; red, TAMRA-S1PS5). **(B)** Quantification of peptide internalization based on TAMRA fluorescence intensity. **(C)** Stability assay of linear and cyclic S1PS5 peptides in 25% FBS. Residual peptide amount is expressed as the percentage of the initial amount versus time. Results from n=3 independent experiments.

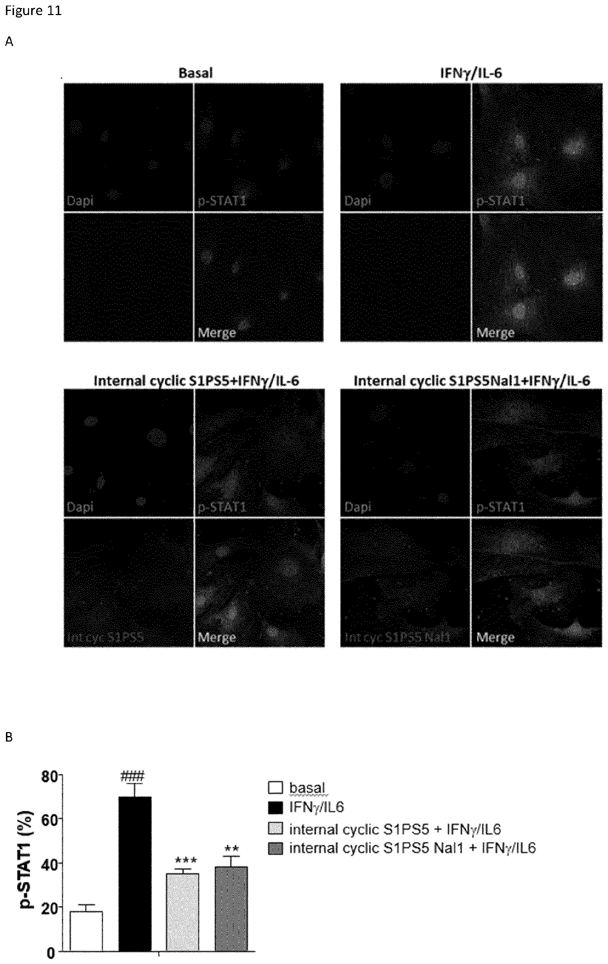

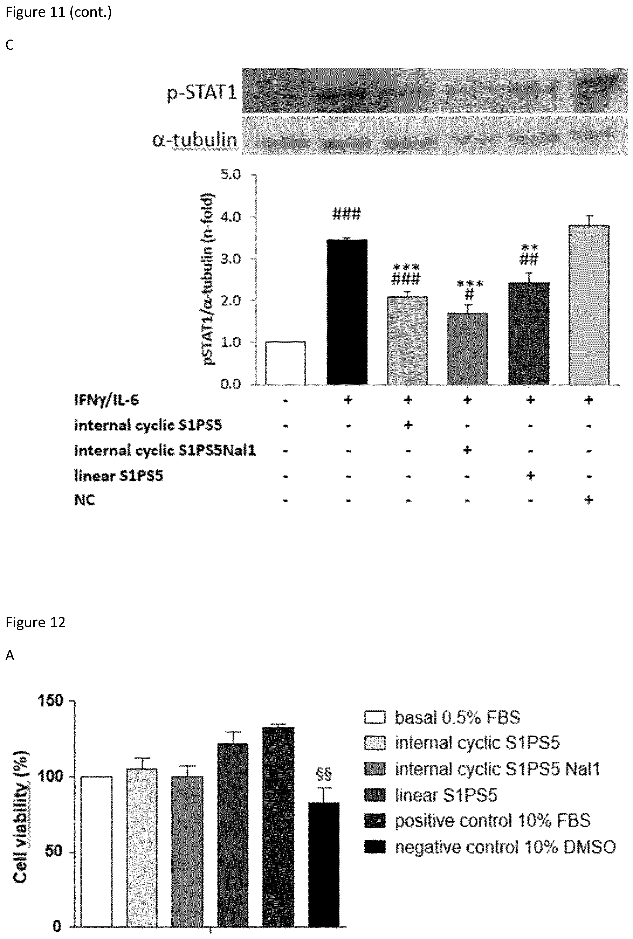

**Figure 11****. Inhibition of STAT phosphorylation by cyclic S1PS5 peptides. (A)** Representative confocal images of VSMCs under basal conditions and stimulated for 60min with a combination of IFNγ plus IL-6 in the absence or presence of TAMRA-internal cyclic S1PS5 and S1PS5Nal1 peptides (blue, cell nuclei; red, TAMRA-S1PS5; green, p-STAT1). **(B)** Summary of the quantification of p-STAT1 positive cells expressed as percentage of total cells. **(C)** Representative immunoblots and summary of densitometric analysis of p-STAT1 and α-tubulin (loading control) in mouse VSMCs incubated with cytokines and S1PS5 peptides and negative control (NC) peptide. Data are expressed as mean±SEM of n=3 independent experiments. ^{#}P<0.05, ^{##}P<0.01, ^{###}P<0.001 vs basal; **P<0.01, ***P<0.001 vs cytokines

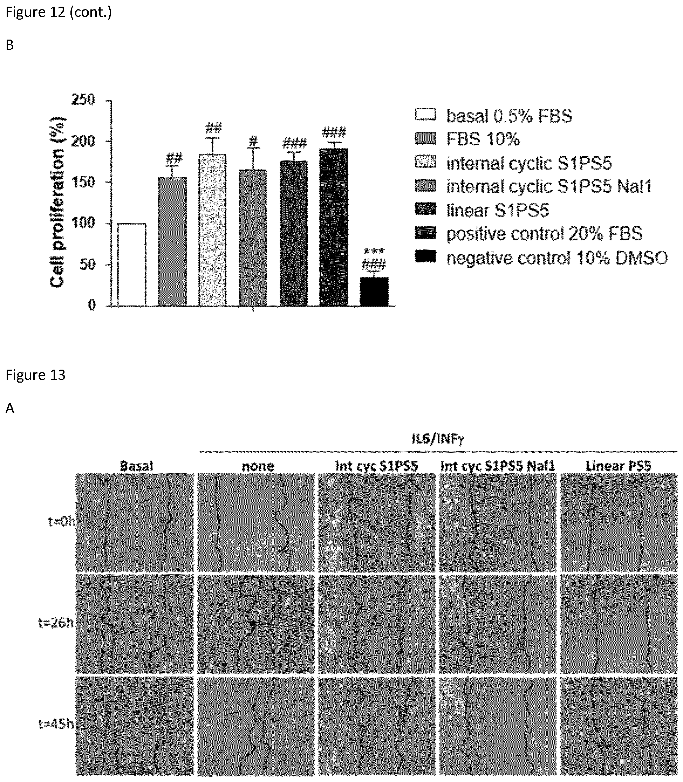

**Figure 12****. Effects of S1PS5 peptides on cell viability and proliferation. (A)** MTT cell viability assay in VSMCs maintained for 24 hours under basal conditions (medium with 0.5% FBS) in the presence or absence of 12.5µM S1PS5 peptides, using 10% FBS and 10%DMSO as positive and negative controls, respectively. Mean±SEM (^{§ §}P<0.01 vs positive control). **(B)** MTT cell proliferation assay in VSMCs maintained for 24 hours under basal conditions or in medium containing 10% FBS with/without S1PS5 peptides, using 20% FBS and 10%DMSO as positive and negative controls, respectively. Mean±SEM (^{#}P<0.05, ^{##}P<0.01, ^{###}P<0.001 vs basal, *P<0.05, ***P<0.001 vs positive control). Results from n=3 independent experiments performed in quadruplicate.

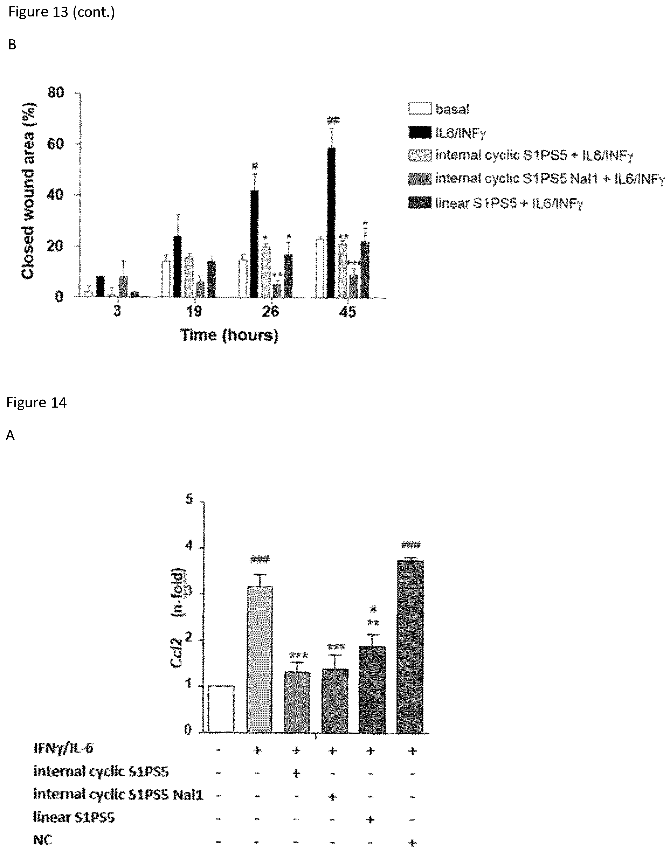

**Figure 13****. Effects of S1PS5 peptides on cell migration. (A)** Representative images of the scratch wound healing assay in mouse VSMCs stimulated with cytokines in the presence or absence of S1PS5 peptides. **(B)** Quantifications of covered healing areas at the indicated times are expressed as percentage of the initial wound area. Mean±SEM of 3 independent experiments. ^{#}P<0.05, ^{##}P<0.01 vs basal; *P<0.05, **P<0.01, ***P<0.001 vs cytokines.

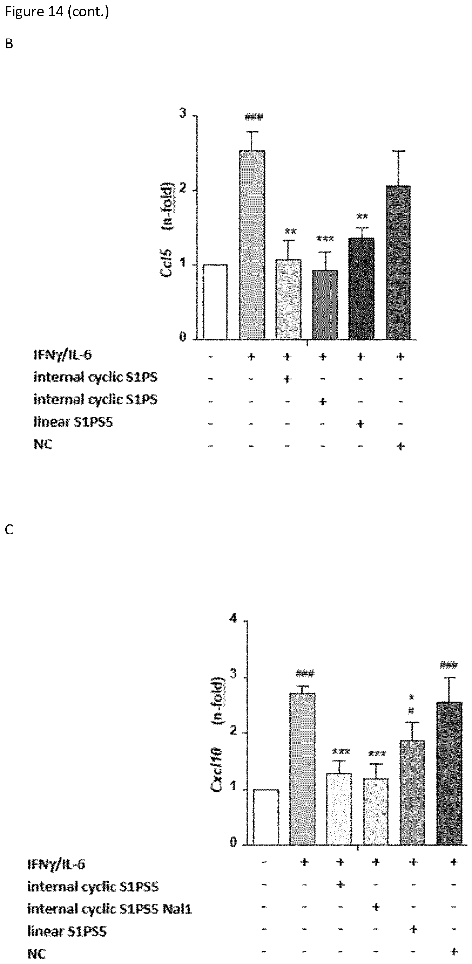

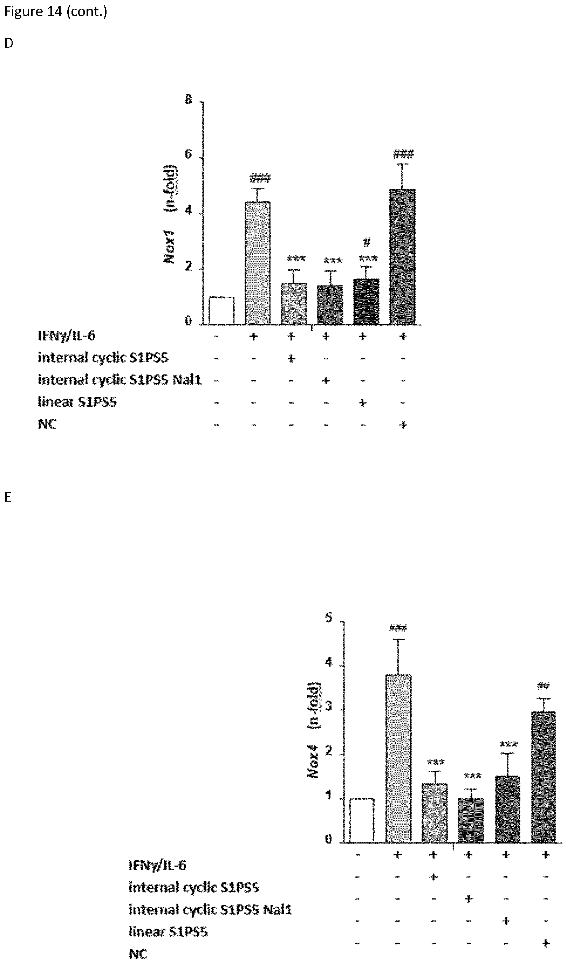

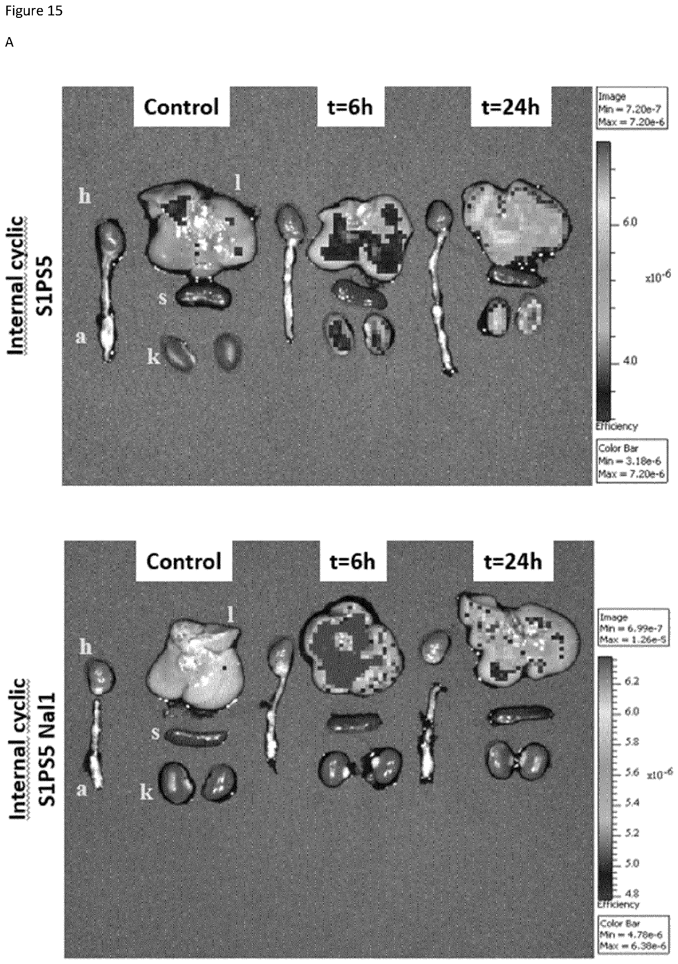

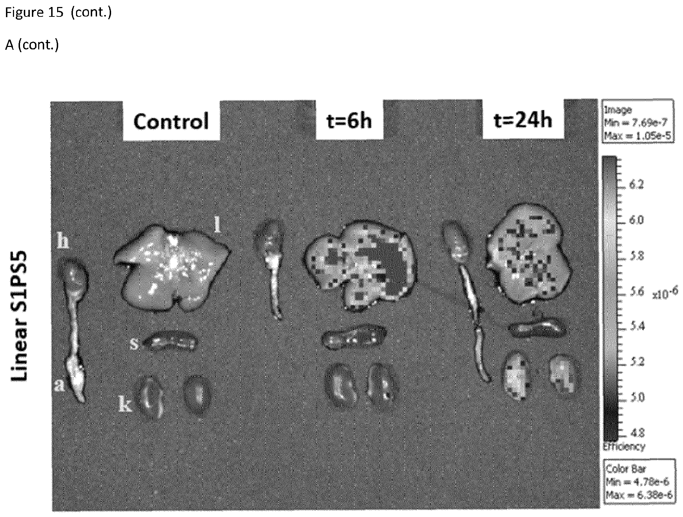

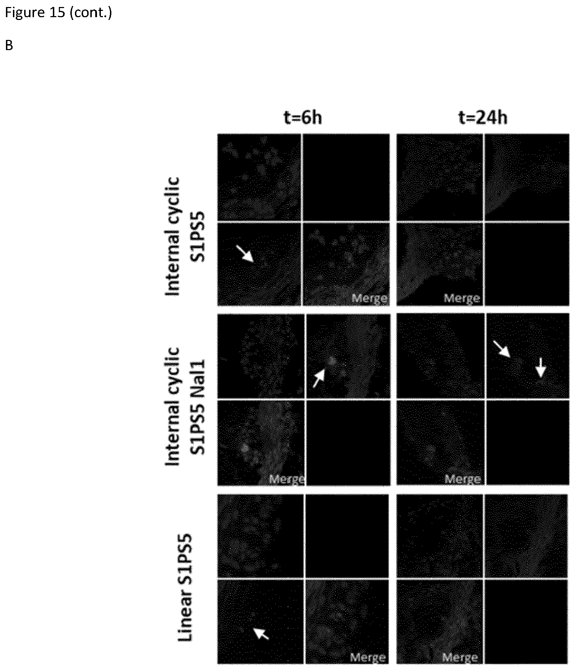

**Figure 14****. Real-time PCR analysis of STAT-dependent genes in mouse VSMCs.** Cells were stimulated with cytokines (IFNγ plus IL-6) for 6 hours in the absence or presence of 12.5µM of S1PS5 peptides and negative control peptide (NC). The mRNA expression levels of *Ccl2* **(A)**, *Ccl5* **(B)**, *Cxcl10* **(C)**, *Nox1* **(D)** and *Nox4* **(E)** were normalized to 18S and expressed as fold increases over basal conditions. Results are the mean±SEM of 3 independent experiments. #P<0.05, ^{##}P<0.01, ^{###}P<0.001 vs basal; *P<0.05, **P<0.01, ***P<0.001 vs cytokines. **Figure 15****. Biodistribution of internal cyclic S1PS5, internal cyclic S1PS5 Nal1 and linear S1PS5 in mice. (A)** *Ex vivo* images showing tissue biodistribution of TAMRA-conjugated peptides after 6 and 24h of intraperitoneal injection. Symbols: h, heart; a, aorta; 1, liver; s, spleen; k, kidney. **(B)** Representative confocal microscopy images in aorta cross-sections showing the localization of fluorescent peptides (arrows) in the aortic lesion (blue, cell nuclei stained with DAPI; red, TAMRA-S1PS5).

### Detailed description of the invention

The present invention is illustrated by means of the examples set below without the intention of limiting its scope of protection.

### Example 1. Material and methods

### Example 1.1. Peptide synthesis

S1 and S1PS5 peptides derived from SOCS1 KIR domain (residues 53-68) conjugated with the cell penetrating sequences at N-terminal were synthesized by solid phase method following the Fmoc strategy, purified by preparative reverse phase-HPLC and lyophilized. Peptides were dissolved in 1% DMSO in saline solution (S1) and H₂O (S1PS5), then filter-sterilized and stored at -80°C before use. For biodistribution studies, peptides were labelled with rhodamine or carboxytetramethylrhodamine (TAMRA) at N-terminal. For stability assays, peptides were incubated at 37°C with 25% foetal bovine serum (FBS). At selected times, 80 µL aliquots were taken, precipitated with 15% trichloroacetic acid, and further analysed by reverse phase HPLC with UV detector.

### Example 1.2. Experimental model of AAA.

All the procedures carried out in this study were performed under the principle for replacement, refinement or reduction (the 3Rs) and in accordance with the Directive 2010/63/EU of the European Parliament and were approved by the Institutional Animal Care and Use Committee of IIS-Fundacion Jimenez Diaz and Comunidad de Madrid (PROEX 116/16 and 217/19). C57BL/6J mice (*Mus musculus*, males, 10-12 weeks old, 23-28 g body weight; Charles River Laboratories, France) were housed in ventilated cages (2-4 mice per cage) with usual bedding material and environmental enrichment in a conventional temperature-controlled room (20-22°C) with 12 h light/dark cycle and free access to water and standard food. Animal studies are reported in compliance with the ARRIVE guidelines and with the recommendations made by the *British Journal of Pharmacology.*

In these studies, we developed the murine elastase model of AAA, which is a well-established and useful approach for characterising the early events in AAA formation (Sénémaud *et al.*, 2017; Yoshimura *et al.*, 2018). AAA was induced in the infrarenal abdominal aorta of mice by intra-aortic perfusion of porcine pancreatic elastase type I (specific activity 7 U.mg⁻¹ protein; E1250; Sigma Chemical Co), as described previously (Fernandez-Garcia *et al.*, 2017; Tarin *et al.*, 2014). Briefly, mice were anaesthetized by 2% isoflurane inhalational anaesthesia and a horizontal laparotomy was performed. Using a surgical stereomicroscope, the abdominal aorta was separated from the level of the left renal vein to the bifurcation and temporarily ligated between the renal and iliac arteries. An aortotomy was created with a 30-gauge needle and the aorta was exsanguinated. A PE-26 polyethylene tube was introduced through the aortotomy, and the aorta was infused for 5 min at 100 mmHg with saline or elastase solution. Aortotomy was then repaired, ligation was eliminated and restoration of blood flow visually confirmed. After suturing, mice received postoperative thermal support and then housed under standard conditions.

Mice were randomly distributed into three groups: 1) Sham operation (saline perfusion); 2) AAA control (elastase perfusion and vehicle administration, 0.1% DMSO); and 3) AAA plus S1 peptide treatment (elastase perfusion and SOCS1-derived peptide administration, 4 mg.kg⁻¹ body weight, intraperitoneal, 3 days/week). Treatments were started 1 day before operation. On the day 14^{th} post-surgery, mice were anaesthetized (ketamine 100 mg.kg⁻¹ and xylazine 10 mg.kg⁻¹) and euthanized, and blood and tissue samples were then collected. The type and number of aortic samples processed were: histology (abdominal aorta: Sham, n=10; Control, n=12; S1, n=12), mRNA expression (abdominal aorta: Sham, n=9; abdominal and thoracic aorta: Control, n=10; S1, n=9) and protein expression (abdominal aorta: Control, n=6; S1, n=6). For biodistribution experiments, on the day 7^{th} after elastase perfusion mice received a single intraperitoneal injection of rhodamine-labelled SOCS1 peptide or vehicle (n=3). At 3, 6, 18- and 24-hours post-injection, mice were anaesthetized with isoflurane and observed by an *in vivo* imaging system (IVIS-Lumina; Caliper Life Sciences Inc., Hopkinton, MA) coupled with Living Image software (Xenogen Corporation, Alameda, CA). *Ex vivo* images of dissected tissues including liver, spleen, kidney, heart and aorta were also immediately recorded with IVIS spectrum.

### Example 1.3. Histological and immunohistochemical analysis.

At the time of harvest, aortic samples were fixed in 10% buffered formalin and processed using routine paraffin embedding. Following deparaffinization, aortic tissue cross-sections (3-4 µm) were stained with Masson's trichrome and Verhoeff-van Gieson. Changes in aortic diameter were calculated as percentage of dilation over baseline at day 14. For immunohistochemistry, aortic tissue sections were deparaffinized and dehydrated through graded xylene and ethanol. After antigen retrieval (0.01 M citrate buffer pH 6 for 20 min) and blockade of endogenous peroxidase (3% H₂O₂ in methanol for 30 min) and nonspecific binding (8% host serum for 30 min), slides were incubated overnight at 4°C with primary antibodies against phosphorylated STAT1 (p-STAT1; Thermo Fisher Scientific Cat# 44-376G, RRID:AB_2533642), p-STAT3 (Cell Signalling Technology Cat# 9134, RRID:AB_331589), calponin (Abcam Cat# ab46794, RRID:AB_2291941), F4/80 (Bio-Rad Cat# MCA497R, RRID:AB_323279), CD68 (Abcam Cat# ab53444, RRID:AB_869007), Ly6G (BioLegend Cat# 108402, RRID:AB_313367), CD3 (Agilent Cat# A0452, RRID:AB_2335677), CD45R (BD Biosciences Cat# 550286, RRID:AB_393581), and 8-hydroxy-2'-deoxyguanosine (8OHdG; Abcam Cat# ab10802, RRID:AB_297482). After rinsing in PBS, samples were incubated with biotinylated secondary antibodies, followed by avidin-biotin complex reagent (Vector Laboratories). Immunoreactive cells were then visualized by the addition of peroxidase substrates (3, 3-diaminobenzidine or 3-amino-9-ethylcarbazole; DAKO) and counterstained with hematoxylin. VSMC content was also analysed by direct immunofluorescence with α-smooth muscle actin (a-SMA) antibody (Agilent Cat# M0851, RRID:AB_2223500). Intracellular superoxide anion was assessed by microscopy using the sensitive fluorescent dye dihydroethidium (DHE; Molecular Probes) (Lopez-Sanz *et al.*, 2018). All the histological evaluations were conducted in a blinded fashion. Elastin fragmentation and VSMC content in the media were graded (score 0-4) as previously described (Fernandez-Garcia *et al.*, 2017; Tarin *et al.*, 2014). Positive staining was quantified in at least 2 sections per mice using Image-Pro Plus software (Media Cybernetics, Bethesda, MD) and expressed as percentage or number of positive cells per lesion area.

### Example 1.4. Cell cultures.

Primary VSMC were isolated from mouse aorta by enzymatic digestion with collagenase type II (C6885, Sigma-Aldrich), cultured in Dulbecco's Modified Eagle Medium (DMEM; D6546, Sigma-Aldrich) containing 10% FBS, 100 U.mL⁻¹ penicillin, 100 µg.mL⁻¹ streptomycin and 2 mM L-glutamine (Sigma-Aldrich), and used between 2^{nd}-8^{th} passages as previously reported (Ortiz-Munoz *et al.*, 2009; Recio *et al.*, 2015). Mouse VSMC line MOVAS-1 (ATCC Cat# CRL-2797, RRID:CVCL_0F08) was maintained in DMEM supplemented with 10% FBS, antibiotics, L-glutamine and 0.2 mg.mL⁻¹ G-418. Mouse macrophage cell line RAW 264.7 (ATCC Cat# TIB-71, RRID:CVCL_0493) was maintained in DMEM with 10% FBS. Murine bone marrow-derived macrophages (BMDM) were obtained after 7 days in DMEM containing 10% FBS and 20% L929 cell-conditioned medium as a source of macrophage colony stimulating factor (Mallavia *et al.*, 2013). Cells were made quiescent by 24 hours incubation in medium with 0.5% FBS, then treated with different doses of S1 and S1PS5 peptides for 90 min before stimulation with elastase (0.7-5 µg.mL⁻¹), elastin-derived hexapeptide VGVAPG or scramble control peptide VVGPGA (0.1-10 µg.mL⁻¹; Proteogenix) for additional 6-24 hours. Cell viability and proliferation were determined in quadruplicate by the 3-(4,5-dimethylthiazol-2-yl)-2,5-diphenyltetrazolium bromide tetrazolium (MTT) assay using medium with 10-20% FBS as positive control, and 10% DMSO as negative control. The absorbance values were used to calculate the percentage of cell viability versus basal conditions.

For gene silencing experiments, VSMC at 60-80% confluency was transfected in Opti-MEM medium with 20-30 nmol of SOCS1 Silencer Select small interfering RNA (siRNA) (Thermo Fisher Scientific Cat# s63995) or Silencer Select Negative Control siRNA (Thermo Fisher Scientific Cat#4390843) using Lipofectamine RNAiMAX Transfection Reagent (Thermo Fisher Scientific Cat#13778075). Twenty-four hours after transfection, cells were incubated with elastase and S1 peptide for additional 24 hours, and then processed for Western blot and real-time PCR analysis.

### Example 1.5. Real time PCR analysis.

Total mRNA from mouse aorta and cultured cells was extracted using TRI Reagent (Molecular Research Center). Complementary DNA was produced via reverse transcription using High Capacity cDNA Reverse Transcription Kit (Applied Biosystems). The gene expression of mouse *Socs1, Socs3, Stat1, Stat3, Ccl2, Ccl5, Ifny, Tnfα, Il-10, Il-17*, Intercellular adhesion molecule-1 (*Icaml*), Arginase-1 (*Argl*), *Arg2*, Inducible nitric oxide synthase (*Inos*), Macrophage mannose receptor-1 (*Cd206*), Krueppel-like factor-4 (*Klf4*), Actin-α2 (*Acta2*), Smooth muscle protein 22-α (*Sm22α*), *Mmp2*, *Mmp9*, Tissue inhibitor of metalloproteinase-1 (*Timp1*), *Timp2*, C-X-C motif chemokine ligand 10 (*Cxcl10*) and NADPH oxidase subunits (*Nox1*, *Nox2* and *Nox4*) was determined in duplicate by quantitative real-time PCR using commercial primers and Premix Ex Taq (Takara). The results for each sample were normalised to the 18S housekeeping expression, and data expressed in arbitrary units (*in vivo* studies) or converted to fold increases versus basal conditions (*in vitro* studies). The online database STRING (http://string-db.org) was used to construct the protein-protein interaction network for the genes analysed.

### Example 1.6. Protein expression analysis.

Cells were lysed in 10 mM Tris pH 7.4, 150 mM NaCl containing 1% Triton X-100, 0.5% NP-40, 1 mM EDTA, 1 mM EGTA, 0.2 mM Na₃VO₄, 10 mM NaF, 0.2 mM PMSF, and protease inhibitor cocktail. Total proteins (20 µg) were electrophoresed and immunoblotted for p-STAT1 (Innovative Research Cat# 33-3400, RRID:AB_87095), p-STAT3 (Cell Signalling Technology Cat# 9134, RRID:AB_331589), STAT1 (Cell Signalling Technology Cat# 9172, RRID:AB_2198300), STAT3 (Cell Signalling Technology Cat# 9139, RRID:AB_331757) and β-tubulin (Sigma-Aldrich Cat# T5168, RRID:AB_477579). Densitometric data were normalised to loading control and expressed in arbitrary units (*in vivo* studies) or converted to fold increases versus basal conditions (*in vitro* studies). CCL2 chemokine levels in cell supernatants were determined in duplicate by ELISA (R&D Systems).

### Example 1.7. Gelatin zymography for MMPs.

The MMP detection in mouse sera and cell supernatants was assessed by gel-based gelatine zymography. The cell culture supernatants were collected, centrifuged for removing debris, and concentrated 4-6 times with a 10 kDa microcon ultrafiltration device (Millipore). Equal amounts of either mouse serum samples or cell supernatants were mixed with sample buffer and electrophoresed onto 10% SDS-PAGE gels containing 1 mg.mL⁻¹ gelatine (Novex Zymogram Plus Gels, ThermoFisher Scientific). After electrophoresis, the gels were rinsed 3 times for 30 min with washing buffer containing 2.5% Triton X-100, 30 min with H₂O, and then incubated at 37°C overnight in 50 mM Tris-HCl pH 7.5 containing 200 mM NaCl and 100 mM CaCl₂. The gels were stained with 0.5% Coomassie brilliant blue R-250 solution containing 10% acetic acid and 20% methanol for 30 min. Areas of gelatinase activity were visualized as clear bands against a blue background. Densitometry values of active MMP bands were converted to fold changes versus Sham group (*in vivo* studies) or basal conditions (*in vitro* studies) for representation.

### Example 1.8. In vitro wound healing assay.

VSMC migration was measured by the wound healing assay as previously described (Recio *et al.*, 2014). Briefly, cell monolayers of MOVAS-1 cell line on 12-well multiplates were cultured until 90-95% confluence, and then scratches were applied using a sterile 200-µl pipette tip. After washing in PBS, the initial wounds were examined under phase contrast microscope (Nikon). Then, cells were incubated for 0-45 hours in medium containing elastase or cytokines in the presence or absence of S1 and S1PS5 peptides. Images were collected during the healing period, and wound closure areas were measured in quadruplicate and expressed as percentages relative to time 0 values.

### Example 1.9. Statistical analysis.

Results are presented as individual data and mean±SEM from separate animals and cell experiments. In all the analysis, technical replicates were averaged to provide a single value per each biological replicate. Statistical analyses were performed using GraphPad v.5 (GraphPad Software Inc., La Joya, CA). Data passed the D'Agostino and Pearson omnibus normality test and were tested for homogeneity of variance with the Bartlett test. Pearson's correlation analysis was performed for normally distributed parameters. Statistical significance was set at *p<0.05* using unpaired two-tailed Student's t test, one-way ANOVA with Bonferroni pairwise comparison test or two-way ANOVA with Tukey's multiple comparisons test. Results expressed as fold changes versus Sham or basal conditions to avoid unwanted sources of variation, were subjected to non-parametric Mann-Whitney test or Kruskal-Wallis test with by Dunn's multiple comparisons test.

### Example 2. Results

### Example 2.1. Activation of JAK/STAT/SOCS axis in experimental AAA correlates with aneurysm size.

To investigate a direct interaction between JAK/STAT pathway and experimental AAA formation, we performed immunohistochemical analysis and quantitative real-time PCR in abdominal aortic samples from elastase-induced AAA mouse model. The results showed a strong activation/phosphorylation of STAT1 (p-STAT1; **Fig. 1A**) and p-STAT3 (**Fig. 1B**) in AAA lesions colocalizing with F4/80⁺ macrophages. There was a positive linear correlation of p-STAT1/3 levels with aneurysm diameter increase (**Fig. 1C**). In addition, and compared with Sham operated animals, we found an increased mRNA expression of STAT1 (3.2-fold), STAT3 (5.3-fold), SOCS1 (3.1-fold) and SOCS3 (2.5-fold) in abdominal aorta from elastase-perfused mice, and a significant positive correlation between STATs and SOCSs expression levels (**Fig**. **1****, D** and **E**). Pearson's correlation analyses further confirmed that the levels of STAT expression and activation are closely related to different examined parameters in AAA lesion such as inflammatory cell and VSMC content, oxidative stress markers and gene expression of chemokines, cytokines and MMPs (**Table 1**).

**Table 1**

| **1. HISTOLOGICAL ANALYSIS** | | |
|---|---|---|
| **Marker** | **p-STAT1 staining** | **p-STAT3 staining** |
| α-SMA content | 0.5886 (*p*=0.0063) | 0.6449 (*p*=0.0021) |
| Elastin degradation | 0.3518 (ns) | 0.3571 (ns) |
| CD68⁺ macrophages | 0.5758 (*p*=0.0079) | 0.6648 (*p*=0.0014) |
| Ly6G⁺ neutrophils | 0.7054 (*p*=0.0005) | 0.6552 (*p*=0.0017) |
| CD3⁺ T-lymphocytes | 0.6851 (*p*=0.0034) | 0.6966 (*p*=0.0027) |
| CD45R⁺ B-cells | 0.8385 (*p*<0.0001) | 0.8467 (*p*<0.0001) |
| DHE | 0.6527 (*p*=0.0061) | 0.7073 (*p*=0.0022) |
| 8OHdG | 0.6932 (*p*=0.0029) | 0.6964 (*p*=0.0027) |

| 2. REAL-TIME PCR ANALYSIS | | |
|---|---|---|
| Gene | **Stat1** mRNA | Stat3 mRNA |
| *Ccl2* | 0.6180 (*p*=0.0048) | 0.7891 (*p*<0.0001) |
| *Ccl5* | 0.5425 (*p*=0.0164) | 0.5441 (*p*=0.016) |
| *Ifnγ* | 0.5728 (*p*=0.0104) | 0.6454 (*p*=0.0028) |
| *Tnfα* | 0.6902 (*p*=0.0011) | 0.4376 (ns) |
| *Mmp2* | 0.5001 (*p*=0.0292) | 0.6503 (*p*=0.0026) |
| *Mmp9* | 0.6178 (*p*=0.0048) | 0.6593 (*p*=0.0021) |
| *Il-10* | -0.382 (ns) | -0.2997 (ns) |
| *Il-17* | 0.4762 (*p*=0.0393) | 0.6005 (*p*=0.0066) |
| *Icam1* | 0.5976 (*p*=0.0069) | 0.613 (*p*=0.0053) |

| | | |
|---|---|---|
| *Correlations of STAT phosphorylation and mRNA expression levels with histological markers and gene expression in AAA tissue. Pearson's correlation coefficient r (and p values) in the elastase-induced mouse model (n*=*10 animals). ns, non-significant.* | | |

### Example 2.2. SOCS1-derived peptide inhibits JAK/STAT and protects mice from elastase-induced AAA formation.

We next examined the therapeutic effect of S1 peptide when administered to elastase-injured mice. This synthetic, cell-penetrating peptide derives from the KIR domain of SOCS1 that specifically binds to JAK autophosphorylation site and inhibits JAK1/2 and TYK2 kinase activity (Jager *et al.*, 2011). In previous studies, we have determined the structure, pharmacokinetic parameters and effectiveness of S1 in different models (Opazo-Rios *et al.*, 2020; Recio *et al.*, 2017; Recio *et al.*, 2014). The macroscopic biodistribution of fluorescent S1 peptide in elastase-infused mice was evaluated by *in vivo* and *ex vivo* imaging system. Results show efficient fluorescence accumulation in mouse tissues in addition to the injury site of the abdominal aorta, whereas no signal was observed in mice receiving vehicle (**Fig. 2****, A-C**). At the end of the treatment period, the increments in the mRNA expression of STAT1/3 and SOCS1/3 (**Fig. 2D**) observed in abdominal aorta, compared with thoracic aorta of elastase-perfused mice, were all significantly decreased by S1 peptide treatment. Furthermore, significant reductions in STAT1/3 protein expression (**Fig. 2E**) and phosphorylation (**Fig. 2F**) were detected in AAA lesions from mice receiving S1 peptide, as compared to vehicle control group.

Fourteen days after elastase perfusion, histological examination of mouse abdominal segments revealed severe dilation in the aortic lumen compared with Sham operated animals (**Fig.3A**). Remarkably, the incidence of AAA (defined as an increase of 100% over the initial aortic diameter) was substantially reduced in mice treated with S1 peptide compared with control AAA mice receiving vehicle (Incidence rate: Control (12/12) 100%; S1 (6/12) 50%). S1 treatment also significantly decreased the maximal aortic dilation (diameter increase, % versus Control: 47±6; **Fig. 3B**). Aneurysm rupture was not observed in the experimental groups (Control and S1). In addition, Verhoeff-van Gieson staining showed that the elastin fibers were less disrupted in the S1-treated mice compared with the Control group (Fig. 3, A and C). In addition, AAA lesions from S1-treated mice exhibited improved preservation of medial VSMC content, as evidenced by immunodetection of contractile proteins α-SMA and calponin in aortic sections (**Fig. 3D**).

### Example 2.3. SOCS1-derived peptide reduces inflammation and oxidative stress in AAA lesions.

To evaluate whether S1 peptide affects local inflammatory and oxidative stress responses in experimental AAA, aortic samples were analysed for infiltrating leukocytes and ROS content. In comparison with the vehicle control group, treating mice with S1 peptide significantly decreased the accumulation of CD68⁺ macrophages, Ly6G⁺ neutrophils, CD3⁺ T-cells, and CD45R⁺ B-cells in AAA lesions (**Fig. 4****, A-E**). Moreover, aortic levels of superoxide anion and oxidative DNA damage marker 8OHdG (**Fig**. **4A**) were significantly reduced in S1-treated group (% versus Control: 38±9 and 41±6, respectively; **Fig. 4F**). Concomitantly, a reduced mRNA expression of chemokines (*Ccl2* and *Ccl5*) and cytokines (*Ifnγ*, *Tnfα*) was observed in abdominal aorta of SI-treated mice (**Fig**. **5A**), whereas no significant changes were observed in thoracic aorta. S1 therapy also downregulated inflammatory genes (*Il-17* and *Icam1*) and M1 macrophage markers (*Arg2* and *Inos*), along with upregulation of antiinflammatory cytokine (*Il-10*) and M2 markers (*Arg1* and *Cd206*) (**Fig. 5****, B** and **C**). Real-time PCR analysis also revealed a decline in the expression levels of proliferative, synthetic VSMC phenotype marker *(Klf4),* whereas the expression of contractile VSMC genes (*Acta2* and *Sm22α*) was increased (**Fig. 5C**). Lower gene expression of elastolytic enzymes (*Mmp2* and *Mmp9*) was detected in AAA tissue from S1-treated mice compared with controls (**Fig. 5B**). Furthermore, gelatin zymography revealed a decreased intensity of active MMP9 and MMP2 bands in serum samples from S1 treated mice (**Fig. 5D**).

### Example 2.4. SOCS1 modulates cell responses to elastase and elastin-derived peptide.

In an attempt to establish an *in vitro* AAA microenvironment, cultured VSMC and macrophages were exposed to either elastase (2.5 and 0.7 µg.mL⁻¹, respectively) or elastin degradation products, named elastin-derived peptides (EDP, 1 µg.mL⁻¹) for different time periods. These chosen doses were based on previous reports (Ghosh *et al.*, 2014; Salmon *et al.*, 2013), and the MTT assays demonstrated no cytotoxic effects on cells (**Fig. 6A**). In VSMC and RAW264.7 macrophages we observed that elastase increased the protein phosphorylation (**Fig. 6B**) and mRNA expression (**Fig. 6**, **C** and **D**) of STAT1 and STAT3 compared with basal conditions; and this effect was significantly decreased in the presence of S1 peptide. Real-time PCR analysis also revealed that S1 downregulated the expression of inflammatory genes (*Ccl2*, *Ccl5*, *Cxcl10*, and *Tnfα*) and superoxide-generating enzyme NADPH oxidase subunits (*Nox1* and/or *Nox2)* induced by elastase **(Fig**s**. 6**, **C** and **D**) and also suppressed CCL2 chemokine release to the cell culture medium **(****Fig**. **6E**). A similar effect on gene expression profile was observed in primary mouse BMDM pre-treated with S1 peptide before elastase stimulation (Fig. 6F). Moreover, S1 peptide altered the MMP expression patterns in elastase-stimulated VSMC and RAW264.7 cells, as evidenced by decreased gene expression of *Mmp2*, *Mmp9,* and *Timp1* (but not *Timp2*) (**Fig. 7A**) and lower MMP2/9 gelatinolytic activity in cell conditioned media (**Fig. 7B**).

In separate experiments, we demonstrate the protective effect of S1 peptide in cells exposed to EDP, which is a more physiological stimulus involved in initiation and progression of AAA (Le Page *et al.*, 2019). Real time PCR analysis in VSMC and BMDM revealed that EDP, but not the scramble control, increased the gene expression of cytokines and MMPs in a dose- and time-dependent manner (**Fig**. **7**, **C** and **D**). Moreover, all these genes were significantly downregulated by S1 peptide.

We further examined the regulatory role of S1 peptide on cell migration and differentiation. In VSMC, S1 prominently mitigated the migration and proliferation capacity of elastase-treated cells, as evidenced by lower percentage of cell-covered area in the wound-healing assay (Fig. **8A**). Moreover, S1 peptide significantly reduced the expression levels of synthetic phenotype marker (*Klf4)* but did not modify contractile marker (*Acta2* and *Sm22)* (**Fig. 8B**). In RAW264.7 macrophages, S1 reduced the expression of M1 associated markers (*Arg2* and *Inos*), while upregulating anti-inflammatory M2 phenotype genes (*Arg1* and *Cd206*), thus resulting in lower M1/M2 ratio (**Fig. 8B**).

To further explore the direct effect of SOCS1 on cell responses, gene silencing experiments were performed in VSMC using specific siRNA for SOCS1 or control siRNA. Downregulation of SOCS1 gene in siRNA transfected VSMC (**Fig. 8C**) resulted in enhanced expression of chemokines, cytokines and MMPs in response to elastase (**Fig. 8D**), thus confirming the protective role of SOCS1 from detrimental inflammation. These *in vitro* results suggest that both SOCS1 protein and SOCS1 mimetic peptide may be key modulators of cell responses during aneurysm formation. Finally, a schematic protein-protein interactions prediction was delineated using the STRING database **(**F**ig. 8E**), demonstrating the relation of STAT1/3 and the genes analysed in this study.

### Example. 2.5. Linear and cyclic S1PS5 peptides inhibit inflammatory responses in vitro.

In order to improve the kinetic affinity of inhibitory peptide and its stability to protease degradation, several analogues were designed through medicinal-chemistry approaches. In particular, two sequences containing non-natural residues Cys(Acm) and Nal1 (linear S1PS5 and linear S1PS5Nal1) and their corresponding cyclic analogues containing a lactam bridge between side chains of aspartic acid and lysine (internal cyclic S1PS5 and internal cyclic S1PS5Nal1) were structurally and functionally investigated. A series of *in vitro* experiments in mouse VSMCs demonstrated that both linear and cyclic peptides were able to mimic the biological functions of SOCS1 protein.

Confocal microscopy studies using TAMRA-conjugated linear S1PS5 peptide revealed an efficient uptake and cytoplasmic distribution (**Fig. 9A**). In addition, pretreatment of VSMCs with linear S1PS5 peptide prevented the activation (phosphorylation) of STAT1/3 proteins induced by combination stimulation with the inflammatory cytokines IFNγ plus IL-6 (**Fig. 9B**). Similarly, internal cyclic S1PS5 and internal cyclic S1PS5Nal1 peptides were internalised by VSMCs (**Fig. 10A**) and localized in the cytoplasm. Quantification of peptide internalization revealed efficient uptake after ∼2 hours; however, at 24 hours the internal cyclic S1PS5 peptide appeared almost completely degraded while, instead internal cyclic S1PS5Nal1 only for 40 % (**Fig**. **10B**). In parallel with cellular assays, we analysed the stability of S1PS5 peptides in serum. Cyclic peptides were more resistant to serum protease degradation than linear peptide, which showed a 40% degradation rate at 17-20 hours (**Fig. 10C**). Interestingly, after 20 hours the proteolytic degradation level of internal cyclic S1PS5 Nal1 was lower (∼20%) with respect to internal cyclic S1PS5 (∼40%) due to the presence of a non-natural naphthylalanine. This tendency was also confirmed at 42 hours where the residual concentration of internal cyclic S1PS5 Nal1 was still at ∼40%, while the two other peptides appeared almost completely degraded (**Fig. 10C**).

The inhibitory effect of cyclic peptides on JAK/STAT pathway was analysed by confocal microscopy (**Fig. 11****, A** and **B**) and western blot (**Fig. 11C**). The experiments demonstrated that internal cyclic S1PS5 and internal cyclic S1PS5Nal1 effectively suppressed cytokine-induced STAT activation, whereas an unrelated sequence did not show any significant inhibition.

We next explored the impact of S1PS5 peptides on cell viability, proliferation, and migration. MTT assay revealed that peptide treatment did not affect the viability of VSMCs under the experimental conditions used in this study (**Fig 12A**). Moreover, neither cyclic nor linear S1PS5 peptide inhibited the growth of VSMCs in culture medium containing 10% FBS (**Fig. 12B**). *In vitro* wound-healing assay also demonstrated that S1PS5 peptides prevented the VSMC migration induced by cytokine stimulation (**Fig. 13A**). The quantifications of covered healing areas revealed that internal cyclic S1PS5Nal1 exhibited the highest anti-migratory effect (**Fig. 13B**).

Real-time PCR analyses were performed to determine the effect of peptides on the expression of STAT-regulated genes. In cytokine stimulated VSMCs, both linear and cyclic peptides were able to significantly prevent the mRNA expression levels of chemokines such as *Ccl2* (**Fig. 14A**), *Ccl5* (**Fig. 14B**), and *Cxcl10* (**Fig. 14C**). Peptides also suppressed the gene expression of ROS generating enzymes *Nox1* (**Fig. 14D**) and *Nox4* (**Fig. 14E**). These results confirm the anti-inflammatory and antioxidant potential of S1PS5 peptides that have yet to be explored *in vivo.* In this sense, we recently examined the macroscopic and microscopic biodistribution of TAMRA-conjugated S1PS5 peptides in mice. In a pilot experiment, *ex vivo* IVIS bioimaging revealed that fluorescent peptides were primarily localized in hepatic tissue, reached other target organs/tissues, and then cleared through the renal system (**Fig. 15A**). Furthermore, confocal analysis (**Fig. 15B**) revealed an accumulation of labelled S1PS5 peptides in aortic tissue at 6 hours post-injection. Interestingly, at 24 hours only internal cyclic S1PS5 Nail was detected due to its higher resistance to protease degradation with respect to internal cyclic S1PS5 and linear S1PS5.

## Claims

1. Isolated peptide comprising an inhibitory motif of JAK2 consisting of the Formula I
**DT*X₁X₂X₃*T*X₄*R*X₅*H** (I)
wherein:
**D** is Aspartic acid,
**T** is Threonine,
***X₁*** is selected between Histidine or S-acetaminomethyl-L-cysteine (*C_{Acm}*),
***X₂*** is selected between Phenylalanine or Arginine,
***X₃*** is selected between Arginine or Glutamine,
**T** is Threonine,
***X₄*** is Phenylalanine or 1-naphthylalanine (*Nal₁*),
**R** is Arginine,
***X₅*** is selected between Serine or Lysine, with the proviso that when ***X₅*** is Lysine a lactam bridge is formed with the Aspartic acid to create a cyclic peptide, and
**H** is Histidine,
for use in the treatment of abdominal aortic aneurysm after its clinical manifestation.

2. Isolated peptide, for use, according to claim 1, wherein the peptide comprises the inhibitory motif of JAK2 which consists of SEQ ID NO: 1, SEQ ID NO: 2, SEQ ID NO: 3, SEQ ID NO: 4 or SEQ ID NO: 5.

3. Isolated peptide, for use, according to claim 2, wherein the peptide which comprises the inhibitory motif of JAK2 consisting of SEQ ID NO: 1 further comprises an adjacent peptide fragment at the C-terminus consisting of the SEQ ID NO: 6.

4. Isolated peptide, for use, according to claim 3, wherein the peptide comprises the SEQ ID NO: 7.

5. Isolated peptide, for use, according to any of the previous claims, **characterized in that** the peptide further comprises a cell penetrating peptide at the N-terminus.

6. Isolated peptide, for use, according to claim 5, wherein the peptide which comprises the SEQ ID NO: 7, further comprises a cell penetrating peptide consisting of palmitoyl-lysine at the N-terminus.

7. Isolated peptide, for use, according to claim 6, **characterized in that** the peptide consists of the palmitoyl-SEQ ID NO: 8 or a peptide having at least 85% of identity with the SEQ ID NO: 8.

8. Isolated peptide, for use, according to claim 5, wherein the peptide which comprises the inhibitory motif of JAK2 consisting the SEQ ID NO: 2, SEQ ID NO: 3, SEQ ID NO: 4 or SEQ ID NO: 5 further comprises a cell penetrating peptide consisting of SEQ ID NO: 9.

9. Isolated peptide, for use, according to claim 8, **characterized in that** the peptide consists of the SEQ ID NO: 10, SEQ ID NO: 11, SEQ ID NO: 12, SEQ ID NO: 13, or a peptide having at least 85% of identity with the SEQ ID NO: 10, SEQ ID NO: 11, SEQ ID NO: 12 or SEQ ID NO: 13.

10. Isolated peptide, for use, according to any of the previous claims, wherein the peptide is administered by intraperitoneal or subcutaneous administration.

11. Isolated peptide consisting of the SEQ ID NO: 10, SEQ ID NO: 11, SEQ ID NO: 12, SEQ ID NO: 13, or a peptide having at least 85% of identity with the SEQ ID NO: 10, SEQ ID NO: 11, SEQ ID NO: 12 or SEQ ID NO: 13.

12. Isolated peptide, according to claim 11, for use as medicament.

13. Pharmaceutical composition comprising an isolated peptide according to claim 11 and, optionally, pharmaceutically acceptable excipients or carriers.


SEQUENCE LISTING

<110>  Fundación Instituto de Investigación Sanitaria Fundación Jiménez Díaz
(FIIS-FJD)
       Daniela Marasco
       Sara La Manna

<120>  TREATMENT OF ABDOMINAL AORTIC ANEURYSM AFTER ITS CLINICAL
       MANIFESTATION

<130>  905592

<160>  13

<170>  PatentIn version 3.5

<210>  1
<211>  10
<212>  PRT
<213>  Artificial sequence

<220>
<223>  Artificial sequence

<400>  1

Asp Thr His Phe Arg Thr Phe Arg Ser His
1               5                   10


<210>  2
<211>  10
<212>  PRT
<213>  Artificial sequence

<220>
<223>  Artificial sequence



<220>
<221>  MISC_FEATURE
<222>  (3)..(3)
<223>  S-acetaminomethyl-Lcysteine

<400>  2

Asp Thr Xaa Arg Gln Thr Phe Arg Ser His
1               5                   10


<210>  3
<211>  10
<212>  PRT
<213>  Artificial sequence

<220>
<223>  Artificial sequence



<220>
<221>  MISC_FEATURE
<222>  (3)..(3)
<223>  S-acetaminomethyl-Lcysteine

<220>
<221> MISC_FEATURE
<222> (7)..(7)
<223> 1-naphthylalanine

<400> 3

Asp Thr Xaa Arg Gln Thr Xaa Arg Ser His
1               5                   10


<210> 4
<211> 10
<212> PRT
<213> Artificial sequence

<220>
<223> Artificial sequence


<220>
<221> MISC_FEATURE
<222> (3)..(3)
<223> S-acetaminomethyl-Lcysteine

<400> 4

Asp Thr Xaa Arg Gln Thr Phe Arg Lys His
1               5                   10


<210> 5
<211> 10
<212> PRT
<213> Artificial sequence

<220>
<223> Artificial sequence


<220>
<221> MISC_FEATURE
<222> (3)..(3)
<223> S-acetaminomethyl-Lcysteine

<220>
<221> MISC_FEATURE
<222> (7)..(7)
<223> 1-naphthylalanine

<400> 5

Asp Thr Xaa Arg Gln Thr Xaa Arg Lys His
1               5                   10


<210> 6
<211> 6
<212> PRT
<213> Artificial sequence

<220>

<223>  Artificial sequence

<400>  6

Ala Asp Tyr Arg Arg Ile
1               5


<210>  7
<211>  16
<212>  PRT
<213>  Artificial sequence

<220>
<223>  Artificial sequence

<400>  7

Asp Thr His Phe Arg Thr Phe Arg Ser His Ala Asp Tyr Arg Arg Ile
1               5                   10                  15


<210>  8
<211>  17
<212>  PRT
<213>  Artificial sequence

<220>
<223>  Artificial sequence

<400>  8

Lys Asp Thr His Phe Arg Thr Phe Arg Ser His Ala Asp Tyr Arg Arg
1               5                   10                  15


Ile




<210>  9
<211>  15
<212>  PRT
<213>  Artificial sequence

<220>
<223>  Artificial sequence

<400>  9

Gly Arg Lys Lys Arg Arg Gln Arg Arg Arg Pro Pro Gln Gly Gly
1               5                   10                  15


<210>  10
<211>  25
<212>  PRT
<213>  Artificial sequence

<220>
<223>  Artificial sequence

<220>
<221>  MISC_FEATURE
<222>  (18)..(18)
<223>  S-acetaminomethyl-Lcysteine

<400>  10

Gly Arg Lys Lys Arg Arg Gln Arg Arg Arg Pro Pro Gln Gly Gly Asp
1               5                   10                  15

Thr Xaa Arg Gln Thr Phe Arg Ser His
            20                  25

<210>  11
<211>  25
<212>  PRT
<213>  Artificial sequence

<220>
<223>  Artificial sequence

<220>
<221>  MISC_FEATURE
<222>  (18)..(18)
<223>  S-acetaminomethyl-Lcysteine

<220>
<221>  MISC_FEATURE
<222>  (22)..(22)
<223>  1-naphthylalanine

<400>  11

Gly Arg Lys Lys Arg Arg Gln Arg Arg Arg Pro Pro Gln Gly Gly Asp
1               5                   10                  15

Thr Xaa Arg Gln Thr Xaa Arg Ser His
            20                  25

<210>  12
<211>  25
<212>  PRT
<213>  Artificial sequence

<220>
<223>  Artificial sequence

<220>
<221>  MISC_FEATURE
<222>  (18)..(18)
<223>  S-acetaminomethyl-Lcysteine

<400>  12

Gly Arg Lys Lys Arg Arg Gln Arg Arg Arg Pro Pro Gln Gly Gly Asp
1               5                   10                  15

Thr Xaa Arg Gln Thr Phe Arg Lys His
            20                  25

<210>  13
<211>  25
<212>  PRT
<213>  Artificial sequence

<220>
<223>  Artificial sequence

<220>
<221>  MISC_FEATURE
<222>  (18)..(18)
<223>  S-acetaminomethyl-Lcysteine

<220>
<221>  MISC_FEATURE
<222>  (22)..(22)
<223>  1-naphthylalanine

<400>  13

Gly Arg Lys Lys Arg Arg Gln Arg Arg Arg Pro Pro Gln Gly Gly Asp
1               5                   10                  15

Thr Xaa Arg Gln Thr Xaa Arg Lys His
            20                  25